# EUROPEAN PATENT SPECIFICATION

(11) **EP 2 913 675 B1**
(45) Date of publication and mention of the grant of the patent: **12.02.2020**
(21) Application number: 15156756.7
(22) Date of filing: 26.02.2015
(51) Int. Cl.: G01N 33/68

(54) **GP2 isoforms and their use in autoantibody capture**
GP2-Isoformen und deren Verwendung bei der Autoantikörpererfassung
Isoformes GP2 et leur utilisation dans la capture d'auto-anticorps

(30) Priority: 28.02.2014 EP 14157199
(43) Date of publication of application: 02.09.2015
(73) Proprietor: GA Generic Assays GmbH, 15827 Dahlewitz (DE)
(72) Inventor: Roggenbuck, Dirk, 15344 Strausberg (DE)
(74) Representative: Hertin und Partner Rechts- und Patentanwälte PartG mbB

(56) References cited:
- WO-A1-96/17873
- WO-A1-2011/130546
- WO-A2-2008/089756
- Dirk Roggenbuck ET AL: "Crohn's disease specific pancreatic antibodies: clinical and pathophysiological challenges", Clin Chem Lab Med, 14 November 2013 (2013-11-14), pages 483-494, XP055130857, DOI: 10.1515/cclm-2013-0801 Retrieved from the Internet: URL:http://www.degruyter.com/view/j/cclm.2 014.52.issue-4/cclm-2013-0801/cclm-2013-08 01.xml [retrieved on 2014-07-22]
- DIRK ROGGENBUCK ET AL: "Autoantibodies to GP2, the major zymogen granule membrane glycoprotein, are new markers in Crohn's disease", CLINICA CHIMICA ACTA, ELSEVIER BV, AMSTERDAM, NL, vol. 412, no. 9, 21 December 2010 (2010-12-21), pages 718-724, XP028148105, ISSN: 0009-8981, DOI: 10.1016/J.CCA.2010.12.029 [retrieved on 2010-12-31]
- DIMITRIOS P BOGDANOS ET AL: "Pancreatic-specific autoantibodies to glycoprotein 2 mirror disease location and behaviour in younger patients with Crohn's disease", BMC GASTROENTEROLOGY, vol. 12, no. 1, 1 January 2012 (2012-01-01), pages 102-102, XP055130943, ISSN: 1471-230X, DOI: 10.1186/ar2949
- VALENTINA SOMMA ET AL: "The Novel Crohn's Disease Marker Anti-GP2 Antibody Is Associated with Ileocolonic Location of Disease", GASTROENTEROLOGY RESEARCH AND PRACTICE, vol. 8, no. 3, 1 January 2013 (2013-01-01) , pages 180-7, XP055130952, ISSN: 1687-6121, DOI: 10.1007/s13317-012-0041-4
- VALLORANI M ET AL: "P280 Anti glycoprotein-2 antibody in pediatric inflammatory bowel disease and celiac disease: prevalence, diagnostic value and variation at follow-up", JOURNAL OF CROHN'S AND COLITIS, vol. 8, 20 February 2014 (2014-02-20), XP028617533, ISSN: 1873-9946, DOI: 10.1016/S1873-9946(14)60401-3
- BONACI-NIKOLIC BRANKA ET AL: "Autoantibodies to GP2, the major zymogen granule membrane glycoprotein, in patients with gluten-sensitive enteropathy: A possible serological trap", CLINICA CHIMICA ACTA, ELSEVIER BV, AMSTERDAM, NL, vol. 413, no. 7, 16 January 2012 (2012-01-16), pages 822-823, XP028902407, ISSN: 0009-8981, DOI: 10.1016/J.CCA.2012.01.005

## Description

The invention relates to a method for binding or capturing autoantibodies directed to various Glycoprotein 2 (GP2) isoforms. In particular the invention provides an in vitro method for the diagnosis of an autoimmune disorder by the detection of autoantibodies that bind one or more isoforms of GP2. The invention is characterised by the provision of multiple isoforms of GP2 as autoantibody targets and encompasses the practical utilisation of the finding that the isoform specificity of anti-GP2 autoantibodies enables determination of particular autoimmune diseases. The invention also provides a kit developed for carrying out the claimed method. The present invention is useful for determining whether a sample from an individual comprises autoantibodies associated with an autoimmune disease, and for differentiating between multiple autoimmune diseases that exhibit similar symptoms.

### BACKGROUND OF THE INVENTION

GP2 is a membrane glycoprotein of the acinar cells of the pancreas [1]. GP2 has been detected in the brush-border cells of the intestine and as a component of lysosomes or as free, non-membrane-bound peptide in pancreatic juice. Making up 30 to 45% of the overall membrane protein, it represents the main component of the zymogen granule membrane. Together with other secretory pancreatic proteins of the zymogenic granules, such as syncollin, lectin ZG16p, synaptobrevin 2 and other sulfate matrix proteoglycans, GP2 is a component of lipid rafts of the granular membrane, and syncollin interacts with GP2. These complexes, also including other proteins such as ZG46p, form the submembranous matrix.

Glycoprotein 2 (GP2) has been identified as the main autoantigenic target of Crohn's disease (CD)-specific pancreatic antibodies (PAB) [2,3]. Apart from its previously assumed restricted location in the pancreas, recent data have demonstrated that GP2 is also a constituent of micro-fold (M) cells of the follicle-associated epithelium, which appears to have an antimicrobial effect, like its renal homolog uromodulin (Tamm-Horsfall protein) [4,5]. Additionally, emerging evidence indicates that GP2 is over-expressed at the site of intestinal inflammation in patients with CD, and that this molecule modulates innate and adaptive immune responses [2,6,7]

Both CD and celiac disease (CeD) demonstrate inflammation of the intestine. However, the localization of the intestinal destruction and the pathophysiological mechanisms responsible for the induction of these diseases are quite distinct [8,9]. Nevertheless, the inflammatory processes seen in CD and CeD are believed to be exacerbated by or lead to an impairment of the intestinal barrier [10,11]. Growing evidence has been collected to demonstrate that both clinical entities involve the loss of humoral tolerance to self and microbiota antigens [12-15]. It has been shown in the art that loss of tolerance to GP2 is a characteristic feature of intestinal destruction in patients with CD [16-18].

Loss of tolerance to GP2 has been reported in up to 30% of CD patients and to approximately 8-10% of patients with ulcerative colitis (UC), the other major inflammatory bowel disease (IBD) [19-21]. The clinical significance of these autoantibodies has been assessed and seropositivity for anti-GP2 antibodies appears to identify CD patients with ileocolonic location, stenosing behaviour, and early disease onset [16,17,22-24].

CD-related pathogenic autoantibodies (PAB) have been detected in patients with CeD, a chronic small intestinal immune-mediated enteropathy precipitated by exposure to dietary gluten in genetically predisposed individuals [25,26]. Exposure to gluten in these patients triggers inflammatory processes leading to a variable degree of intestinal damage which is reversible with the initiation of gluten-free diet (GFD). The destructive mucosal changes detected in duodenal and jejunal biopsies lead to villous atrophy with hyperplasia of the crypts, a raised intraepithelial lymphocyte count, and an impairment of the intestinal barrier, a clinical complication also seen in patients with IBD [27,28]. In contrast to the mucosal inflammatory changes in CeD, the transmural inflammation in CD covers all layers of the bowel wall and adventitia and can occur throughout the intestinal tract [29]. Severe tissue lesions such as fissures, abscesses, strictures, and fistulas can develop in the course of CD.

The immunopathogenesis of inflammatory bowel disease (IBD) as well as that of CeD are poorly understood [30,31]. Antigen-driven mechanisms of immunological breakdown operate in both conditions, but it is still unclear whether the loss of tolerance to GP2 can also be seen in a sub-group of patients with CeD. If this feature is present, it could further indicate that an anti-GP2 response is initiated due to the damage of the intestinal barrier and the leaky gut [32].

Two variants of GP2 have been described in 2000, which are produced in the humans due to alternative splicing [33]. In addition to the large form of GP2, containing 527 amino acids and termed alpha, a shorter beta form exists which comprises only 380 amino acids. The beta from seems to be dominantly expressed in human pancreatic tissue.

Currently, four isoforms of GP2 have been described (see tables 1 to 3 of the detailed description of the invention).

Although, according to a number of authors, the level of GP2 and the severity of IBD correlate, the physiological context is unknown. Furthermore, the physiological function of the four known isoforms of GP2 is still unclear.

Peptides having sequences highly similar to the large α-GP2 isoform are said to be responsible for pancreatic tumor formation. Antibodies to GP2 as analyte and marker are intended for use in diagnosing pancreatic cancer and the peptide and its nucleic acid sequence for use in immune therapy of cancerous diseases of the pancreas (WO 01/94409). Antibodies to the small β-GP2 isoform find use as markers of pancreatitis (WO 96/17873). An increase in β-GP2 concentration is said to be indicative of the disease.

Celiac disease (or known as coeliac disease or celiac sprue) is an autoimmune disorder of the small intestine that occurs in people of all ages from infancy onward. Symptoms include pain and discomfort in the digestive tract, chronic constipation and diarrhoea, anaemia and fatigue, but these may be absent, and symptoms in other organ systems have been described.

Diagnosis of CeD can be carried out via multiple approaches, although none are considered entirely reliable. Serological blood tests are the first-line investigation required to make a diagnosis of CeD. Antiendomysial antibodies of the immunoglobulin A (IgA) type can detect CeD. Serology for anti-tTG antibodies may also be applied, whereby current anti-tTG assays rely on a human recombinant protein as an antigen.

CD and UC represent the two most important IBD. They are characterized by chronic, relapsing tissue-destroying inflammatory processes in the digestive system. To date, etiology and pathogenesis of CD as well as UC are unclear. While inflammation in UC predominantly appears in the mucosa and submucosa of colon and rectum, CD is characterized by wall-penetrating, granulomatous inflammatory processes of the entire gastrointestinal tract.

Highly complex and comparatively cost-intensive histological investigations of mucosa biopsies constitute common means in IBD (CD/UC) diagnostics. To this end, biopsies are collected especially from macroscopically conspicuous as well as inconspicuous areas. To efficiently utilize the potential of histopathological differential diagnostics it is, however, necessary to collect biopsies from at least five different anatomic segments of the entire colon, including the rectum, from the terminal ileum and upper gastrointestinal tract. Such analyses are time intensive and invasive, providing significant discomfort to the patient.

Straightforward diagnostic approaches for diagnosis of CeD, CD and UC remain elusive. Although some immunological assays have been developed, additional histological or biopsy-based analyses are often required. Autoantibodies against cytoskeletal proteins have been described in CD patients confirmed by means of biopsy. Autoantibodies against cytokeratin 18, actin, vimentin, desmin and tropomyosin have been found among others. Although cytokeratin 18 autoantibodies have been found to correlate with the activity of the disease, they failed to gain acceptance in IBD routine diagnostics, probably as a result of their low specificity. Explicit reference has been made to the necessary - still to be found - identification of the pancreatic autoantigen(s) in order to clarify the status of autoimmune processes in the pathogenesis of CD and support discrimination of unclear IBD cases by appropriate laboratory diagnostics.

GP2 has been identified previously as a biomarker for pancreatitis and antibodies directed against GP2 have been developed for interrogating GP2 levels in patients with IBD (WO 96/17873 A1). Autoantibody-based diagnostics involving GP2 as an autoantigen have been described previously (WO 2008/089756 A2). Anti-GP2 autoantibodies have been described in some patients with CeD (Bonaci-Nikolic Branka et al, Clinica Chimica Acta 413 (2012) 822-823). However, no isoform-specificity of the autoantibodies has been disclosed previously and differentiation between CD and CeD has been neither disclosed, nor is possible, based on the methods disclosed in the art.

Despite the various assays available for CD, UC or CeD diagnosis, there is still significant uncertainty regarding which approach is ideal.

Furthermore, due to the overlapping symptoms between each of these diseases, most molecular and histological assays are still considered sub-optimal, if not entirely unable to distinguish between separate autoimmune disorders of the gastrointestinal tract. For example, WO 2011/130546 A1 describes a method for distinguishing CD from other autoimmune conditions based on a composite microbial antibody score, which requires a complex analysis in order to enable identification of the conditions. Effective and straightforward molecular diagnostic means are required that effectively provide differentiation between each of the conditions via immunological assays.

### SUMMARY OF THE INVENTION

The invention therefore relates to an in vitro method for the diagnosis of primary sclerosing cholangitis by detection of autoantibodies from a sample that bind to one or more isoforms of Glycoprotein 2 (GP2), comprising
- providing a sample of a subject exhibiting symptoms and/or suspected of having primary sclerosing cholangitis,
- providing two or more isoforms of Glycoprotein 2 (GP2), wherein at least one of isoforms 1 and/or 2 and at least one of isoforms 3 and/or 4 are provided, wherein the amino acid sequences of isoforms 1, 2, 3 and 4 are according to SEQ ID NO 1, 2, 3 and 4, respectively, or amino acid sequences of more than 80%, more than 85%, more than 90% or more preferably more than 95% sequence identity to sequences of SEQ ID NO 1, 2, 3 and 4,
- contacting said sample with said GP2 isoforms, and
- detecting autoantibodies from said sample that bind to said one or more isoforms
- wherein the contacting and detecting steps comprise: allowing autoantibodies in said sample to bind to more than one of said GP2 isoforms, thereby forming a complex (GP2-autoantibody complex), contacting said complex with a label to form a labeled complex; and detecting the presence or absence of the labeled complex for said GP2 isoforms, and associating the presence of the detected autoantibodies in the sample with the presence of primary sclerosing cholangitis.

The invention relates to the surprising and unexpected finding that different isoforms of the GP2 protein are targets for autoantibodies that are associated with different autoimmune diseases.

The various GP2 isoforms, preferably according to those sequences described herein, may therefore be used in the diagnosis and/or differentiation of autoimmune disease, in particular autoimmune disorders associated with autoantibodies that bind components of the digestive or intestinal (gastrointestinal) tract of said subject.

According to the present invention the components of the gastrointestinal tract, to which autoantibodies may bind, include, but are not limited to, the mucosa of the small intestine or other small-bowel tissue, the villous extracellular matrix, intestinal epithelial cells, in particular villous epithelial cells, the endomysium or other tissues or cells of the stomach, small intestine, and colon, in particular the cells lining of the stomach, small intestine, and colon.

It was at the time of the invention entirely unknown that the various isoforms of GP2 could be used as an epitope or target to distinguish between the presence or absence of different autoimmune diseases, preferably those characterised by autoantibodies that bind components of the gastrointestinal tract of a subject.

The method thereby allows differentiation between such diseases on the basis of their distinct autoantibody profiles, which target only a subset of the GP2 isoforms provided herein. The use of multiple GP2 isoforms thereby represents a novel and inventive concept in light of the prior art with respect to the diagnosis of autoimmune diseases using GP2 as a target.

The use of multiple GP2 isoforms as autoantibody targets in diagnostics is common to preferred embodiments of the invention, thereby representing a unifying concept that is novel and unexpected in light of the cited art.

According to the invention, the isoforms are selected from proteins comprising or consisting of:
- amino acid sequences of isoforms 1, 2, 3 and/or 4 of SEQ ID NO 1, 2, 3 and/or 4, respectively, or
- amino acid sequences of more than 80%, more than 85%, more than 90% or more preferably more than 95% sequence identity to SEQ ID NO 1, 2, 3 and/or 4.

The isoforms of GP2 also relate to those of substantially the same amino acid sequence as those explicitly listed. This refers to one or more amino acid sequence that is similar, but not identical to, the amino acid sequence provided explicitly herein.

Variation in length of the amino acid sequences and encoding nucleic acids as described herein is also encompassed by the present invention. A skilled person is capable of providing artificial amino acid sequence variants that are longer or shorter than the specific sequences of SEQ ID NO 1 to 4, which will still exhibit sufficient similarity to the natural forms in order to provide the diagnostic outcomes described herein. For example, shorter variants of the longer isoforms (SEQ ID NO 1 or 2) comprising 10, 20, 30, 40 or 50 amino acids less than the full length form may also enable effective diagnostic outcomes, as described herein. For example, longer variants of the shorter isoforms (SEQ ID NO 3 or 4) comprising 10, 20, 30, 40 or 50 amino acids of GP2 sequence more than the natural length form may also enable effective diagnostic outcomes, as described herein.

Also disclosed herein is a method for the diagnosis of Celiac disease (CeD), wherein the presence of IgG and/or IgA autoantibodies from a sample of said subject that bind to isoforms 1 and/or 2 of GP2 (SEQ ID NO 1 and/or 2) indicates the presence of CeD. This effect is preferably specific to the isoforms 1 and 2. The provision of at least two isoforms, at least one of 3 and/or 4 and at least one of 1 and/or 2, enables more sound CeD diagnosis than was previously possible. This embodiment is therefore also characterized by the unexpected findings related to isoform-specificity of the anti-GP2 autoantibodies.

Disclosed herein is a method for the diagnosis of Celiac disease (CeD), wherein an increased or larger amount of IgG and/or IgA autoantibodies that bind isoforms 1 and/or 2 of GP2 (SEQ ID NO 1 and/or 2) compared to IgG and/or IgA autoantibodies that bind isoforms 3 and/or 4 (SEQ ID NO 3 and/or 4) in a sample of a subject indicates the presence of CeD in said subject.

Also disclosed herein is a method that comprises:
- measuring an amount of IgG and/or IgA autoantibodies that bind isoforms 1 and/or 2 of GP2 (SEQ ID NO 1 and/or 2) and an amount of IgG and/or IgA autoantibodies that bind isoforms 3 and/or 4 (SEQ ID NO 3 and/or 4) in the sample;
- comparing the amount of IgG and/or IgA autoantibodies that bind isoforms 1 and/or 2 of GP2 (SEQ ID NO 1 and/or 2) with the amount of IgG and/or IgA autoantibodies that bind isoforms 3 and/or 4 (SEQ ID NO 3 and/or 4), wherein when the amount of IgG and/or IgA autoantibodies that bind isoforms 1 and/or 2 of GP2 (SEQ ID NO 1 and/or 2) is higher than the amount of IgG and/or IgA autoantibodies that bind isoforms 3 and/or 4 (SEQ ID NO 3 and/or 4) the subject is diagnosed with Celiac disease.

Disclosed herein is a method for the diagnosis of Crohn's disease (CD), wherein the presence of IgG and/or IgA autoantibodies from a sample of said subject that bind to isoforms 1, 2, 3 and/or 4 of GP2 (SEQ ID NO 1, 2, 3 and/or 4), preferably isoforms 2, 3 and/or 4 (SEQ ID NO 2, 3 and/or 4), more preferably isoforms 3 and/or 4 (SEQ ID NO 3 and/or 4), indicates the presence of CD.

It has been shown for the first time that autoantibodies in patients with CD that bind GP2 bind the comparatively shorter forms of GP2, namely isoforms 3 and/or 4. This embodiment is therefore characterized by the unexpected findings related to isoform-specificity of the anti-GP2 autoantibodies.

Also disclosed herein is a method for the diagnosis of Crohn's disease (CD), wherein an increased or larger amount of IgG and/or IgA autoantibodies that bind isoforms 3 and/or 4 of GP2 (SEQ ID NO 3 and/or 4) compared to IgG and/or IgA autoantibodies that bind isoforms 1 and/or 2 (SEQ ID NO 1 and/or 2) in a sample of said subject indicates the presence of CD in said subject.

Also disclosed herein is a method that comprises:
- measuring an amount of IgG and/or IgA autoantibodies that bind isoforms 1 and/or 2 of GP2 (SEQ ID NO 1 and/or 2) and an amount of IgG and/or IgA autoantibodies that bind isoforms 3 and/or 4 (SEQ ID NO 3 and/or 4) in the sample;
- comparing the amount of IgG and/or IgA autoantibodies that bind isoforms 1 and/or 2 of GP2 (SEQ ID NO 1 and/or 2) with the amount of IgG and/or IgA autoantibodies that bind isoforms 3 and/or 4 (SEQ ID NO 3 and/or 4), wherein when the amount of IgG and/or IgA autoantibodies that bind isoforms 3 and/or 4 of GP2 is higher than the amount of IgG and/or IgA autoantibodies that bind isoforms 1 and/or 2 the subject is diagnosed with Crohn's disease.

The method also relates to a method as essentially described herein, whereby said method may be described as an in vitro method for the detection of autoantibodies from a sample that bind to one or more isoforms of Glycoprotein 2 (GP2), comprising one or more of the features described herein.

The provision of the sample to be analysed may relate to either obtaining a sample from a patient, or providing a pre-prepared sample already having been obtained, preferably from a patient exhibiting symptoms and/or suspecting of having an autoimmune disorder, preferably an autoimmune disorder associated with autoantibodies that bind components of the digestive or intestinal tract of said subject.

Examples of the symptoms of said disorders are provided herein and are not intended to limit the scope of the invention. Such symptoms are well-known to skilled practitioners in the field. Any reference to the provision of multiple isoforms comprises the provision of more than one isoform for analysis. The multiple isoforms may be used in the method as described either simultaneously, one after the other, also at different time points during various diagnostic procedures, for example minutes, hours, weeks or months apart. In some embodiments of the invention one isoform alone may be used, for example in follow up analyses for confirmation. The use of multiple isoforms preferably relates to the simultaneous use of multiple isoforms, for example when the isoforms are attached to a single solid phase for analysis with a single sample, or on separate solid phases for analysis of a single sample at the same time (in other words under the same conditions).

The sample of the present invention relates preferably to a sample obtained from a patient, such as a bodily fluid, preferably a blood, plasma or serum sample, but may also relate to stool sample. Tissue samples may also be used in the method of the invention. Any particular processing of the sample is not intended to be limiting to the scope of the invention, essentially any given sample obtained from the patient may be used, with or without additional processing steps before administration in the method described herein.

The contacting of a sample to the GP2 isoforms may take place in any given setting. In one embodiment, a solid phase, to which the isoforms are attached, is used. The sample is preferably provided as a liquid sample and is brought into contact with the GP2 isoforms, thereby allowing the autoantibodies of the sample to interact with the GP2 isoforms under conditions that allow binding of said antibodies to the GP2 epitope. Such conditions are known to a skilled person and may represent biological conditions, in which the relevant proteins are capable of forming their native or near-native structures, in order to allow the binding properties of the antibodies to enable interaction with said isoforms.

The contacting and detection steps may in further embodiments be carried out as follows: allowing the antibody to bind the one or more GP2 isoforms, thereby forming a complex (GP2-autoantibody complex), contacting the complex with a label, such as a labeled indicator antibody, preferably an antibody that binds human immunoglobulin, to form a labeled complex; and detecting the presence or absence of the labeled complex, and preferably associating the presence of the detected antibodies in the sample with the autoimmune disease.

The detection of bound antibodies may be carried out in any given suitable manner, including but not limited to the use of a spectrophotometer to detect colour from a chromogenic substrate, a radiation counter to detect radiation such as a gamma counter for detection of 1251, or a fluorometer to detect fluorescence in the presence of light of a certain wavelength.

Washing of the bound antibodies may be carried out as is commonly known in the art, for example as is carried out in a standard immunoassay, such as an ELISA assay. Additional detection means are described herein.

It was at the time of the invention entirely unknown that the various isoforms of GP2 could be used as an epitope or target to distinguish between the presence or absence of different autoimmune diseases, preferably those characterised by autoantibodies that bind components of the gastrointestinal tract of a subject. The method allows differentiation between such diseases on the basis of their distinct autoantibody profiles, which target only a subset of the GP2 isoforms provided herein. The use of multiple GP2 isoforms thereby represents a novel and inventive concept in light of the prior art with respect to the diagnosis of autoimmune diseases using GP2 as a target.

The method thereby allows the differentiation of autoimmune diseases, which may show very similar disease symptoms with respect to digestive problems, stomach cramps and pain, diarrhea, amongst others, via a simple and cost effective immunoassay, such as an ELISA, thereby avoiding more complicated diagnostic procedures such as endoscopies or biopsy analysis.

In one embodiment the method of the present invention is characterised in that said method provides differentiation of an autoimmune disorder, characterised by autoantibodies that bind components of the gastrointestinal tract of said subject, from one or more other autoimmune disorders also characterised by autoantibodies that bind components of the gastrointestinal tract of said subject.

In one embodiment the method of the present invention is characterised in that said disorder is associated with autoantibodies that bind one or more, but not all, isoforms 1 to 4 of GP2 according to SEQ ID NO 1 to 4. As demonstrated in the experimental examples herein, in some embodiments of the invention the autoantibody populations bind only a subset of the GP2 isoforms, in particular either the long or short forms of the GP2 protein, and not both. For example, the autoantibodies of celiac patients bind only isoforms 1 and 2.

It is disclosed herein that anti-GP2 autoantibodies of CeD patients bind exclusively the isoforms 1 and/or 2 of GP2, whereby isoforms 3 and/or 4 are bound preferably by the autoantibody population of CD patients. The recognition of this fact enables the method as described herein with respect to differentiation between CD and UC, in particular due to the identification of autoantibodies that bind isoform 3 and/or 4, preferably 4, which indicates the presence of CD, and preferably the absence of CeD and/or UC.

Disclosed herein is a method for the diagnosis of diagnosis of Celiac disease (CeD) and differentiation from Crohn's disease (CD), wherein an increased amount of IgG and/or IgA autoantibodies that bind isoforms 1 and/or 2 of GP2 (SEQ ID NO 1 and/or 2) compared to IgG and/or IgA autoantibodies that bind isoforms 3 and/or 4 (SEQ ID NO 3 and/or 4) in a sample of said subject indicates the presence of CeD and the absence of CD in said subject.

Disclosed is also a method for diagnosis of Crohn's disease (CD) and differentiation from Celiac disease (CeD) and/or Ulcerative colitis (UC).

Disclosed herein is a method for the diagnosis of Crohn's disease (CD), wherein the presence of IgG and/or IgA autoantibodies from a sample of said subject that bind to isoforms 3 and/or 4 of GP2 (SEQ ID NO 3 and/or 4) to a comparatively greater extent than to isoforms 1 and/or 2 (SEQ ID NO 1 and/or 2) indicates the presence of CD.

Also disclosed herein is a method for the diagnosis of diagnosis of Crohn's disease (CD) and differentiation from Celiac disease (CeD) and/or Ulcerative colitis (UC), wherein an increased amount of IgG and/or IgA autoantibodies that bind isoforms 3 and/or 4 of GP2 (SEQ ID NO 3 and/or 4) compared to IgG and/or IgA autoantibodies that bind isoforms 1 and/or 2 (SEQ ID NO 1 and/or 2) in a sample of said subject indicates the presence of CD and the absence of CeD and/or UC in said subject.

Disclosed herein is a method for differentiation between Ulcerative colitis (UC) and Crohn's disease (CD), wherein the presence of IgG and/or IgA autoantibodies from a sample of said subject that bind to isoforms 3 and/or 4 of GP2 (SEQ ID NO 3 and/or 4) to a comparatively greater extent than to isoforms 1 and/or 2 (SEQ ID NO 1 and/or 2) indicates the presence of CD, and preferably the absence of CU.

Also disclosed are methods that comprise treatment of the autoimmune disease identified. Potential treatments are mentioned below.

The invention further provides a kit for the diagnosis of an autoimmune disorder by the detection of autoantibodies from a sample that bind to one or more isoforms of Glycoprotein 2 (GP2), comprising two or more isoforms of GP2), wherein at least one of isoforms 1 and/or 2 and at least one of isoforms 3 and/or 4 are present, wherein the amino acid sequences of isoforms 1, 2, 3 and 4 are according to SEQ ID NO 1, 2, 3 and 4, respectively, or amino acid sequences of more than 80%, more than 85%, more than 90% or more preferably more than 95% sequence identity to sequences of SEQ ID NO 1, 2, 3 and 4.

Also disclosed is a a kit for the diagnosis of an autoimmune disorder by the detection of autoantibodies from a sample that bind to one or more isoforms of Glycoprotein 2 (GP2) according to the preceding claim, comprising:
- at least one nucleic acid molecule encoding an isoforms 1 and/or 2 (SEQ ID NO 1 and/or 2) and at least one nucleic acid molecule encoding isoforms 3 and/or 4 (SEQ ID NO 3 and/or 4), such as those according to SEQ ID NO 5 to 8, or a nucleic acid molecule comprising a degenerate sequence thereof, or a complementary sequence thereof, or a sequence of more than 80%, more than 85%, more than 90% or more preferably more than 95% sequence identity to any one or more of SEQ ID NO 5 to 8.

Disclosed herein is the use of the nucleic acid molecules in the method or kit as described herein, wherein said nucleic acid molecules comprise a sequence encoding isoforms 1, 2, 3 and/or 4 (SEQ ID NO 1, 2, 3 and/or 4), such as those according to SEQ ID NO 5 to 8, or a nucleic acid molecule comprising a degenerate sequence thereof, or a complementary sequence thereof, or a sequence of more than 80%, more than 85%, more than 90% or more preferably more than 95% sequence identity to any one or more of SEQ ID NO 5 to 8.

As described herein, the finding that different isoforms of GP2 are targets for autoantibodies that are associated with specific autoimmune diseases, is a surprising and unexpected finding as such.

Therefore the combination of multiple isoforms of GP2 in a format appropriate for carrying out the present method is motivated only by the novel and surprising finding of the present invention. The combination of multiple GP2 isoforms as such is therefore to be considered an unexpected development of the art. There exists no suggestion in the relevant literature that the provision of a kit comprising multiple GP2 isoforms for carrying out the present method should have been developed.

In a preferred embodiment the kit of the present invention is characterised in that said kit comprises:
- amino acid sequences of isoforms 1 and/or 2 (SEQ ID NO 1 and/or 2) and isoforms 3 and/or 4 (SEQ ID NO 3 and/or 4), and
- amino acid sequences of more than 80%, more than 85%, more than 90% or more preferably more than 95% sequence identity to isoforms 1 and/or 2 (SEQ ID NO 1 and/or 2) and isoforms 3 and/or 4 (SEQ ID NO 3 and/or 4).

In one embodiment the kit of the present invention is characterised in that said kit comprises a solid phase to which at least one amino acid sequence of isoforms 1 and/or 2 (SEQ ID NO 1 and/or 2) and at least one amino acid sequence of isoforms 3 and/or 4 (SEQ ID NO 3 and/or 4), or amino acid sequences of more than 80%, more than 85%, more than 90% or more preferably more than 95% sequence identity to isoforms 1 and/or 2 (SEQ ID NO 1 and/or 2) and isoforms 3 and/or 4 (SEQ ID NO 3 and/or 4), are immobilised.

In one embodiment the kit of the present invention comprises additionally:
- one or more human anti-immunoglobulin antibodies, wherein said human anti-immunoglobulin antibodies bind autoantibodies of Ig-subtypes IgG, IgA and/or IgM,
- a label, either capable of binding said human anti-Immunoglobulin antibody, or linked to said anti-Immunoglobulin antibody, and
- means for detecting said label.

The embodiments described herein with reference to the kit of the present invention are intended to also relate to structural features of the components of the method as described herein. The features of the kit as described herein may therefore also be used to characterize the method, and vice versa.

The invention therefore also relates to the use of a kit as described herein for the diagnosis of an autoimmune disorder by the detection of autoantibodies from a sample that bind to at least one amino acid sequence of isoforms 1 and/or 2 (SEQ ID NO 1 and/or 2) or at least one amino acid sequence of isoforms 3 and/or 4 (SEQ ID NO 3 and/or 4).

The invention further relates to a system for the diagnosis of an autoimmune disorder by the detection of autoantibodies from a sample that bind to one or more isoforms of Glycoprotein 2 (GP2), comprising one or more isoforms of GP2, comprising two or more isoforms of GP2), wherein at least one of isoforms 1 and/or 2 and at least one of isoforms 3 and/or 4 are present.

In one embodiment the system comprises: an optionally networked computer processing device configured to perform executable instructions; and a computer program, the computer program comprising a software module executed by the computer processing device to apply a model or algorithm for analyzing said autoantibodies.

In one embodiment the system is characterised in that the computer program further comprises a software module executed by the computer processing device to designate a treatment regimen for the individual.

In one embodiment the system is characterised in that a patient from which said sample has been taken is identified as providing said sample and is optionally treated for the autoimmune disease.

Treatment for an autoimmune disease of the gastrointestinal tract may relate to any appropriate treatment known to a skilled medical practitioner. Medical treatment of IBD may be individualized to each patient. The choice of which drugs to use and by which route to administer them (oral, rectal, injection, infusion) depends on factors including the type, distribution, and severity of the patient's disease, as well as other historical and biochemical prognostic factors, and patient preferences. For example, mesalazine may be administered. Generally, depending on the level of severity, autoimmune IBD may require immunosuppression to control the symptoms, such as prednisone, TNF inhibition, azathioprine (Imuran), methotrexate, or 6-mercaptopurine administration. Often, anti-inflammatory steroids are used to control disease flares. Crohn's disease and ulcerative colitis patients may receive TNF inhibitors. Severe cases may require surgery, such as bowel resection or a temporary or permanent colostomy or ileostomy. Surgery can cure ulcerative colitis if the large intestine is removed. A pouch can be created from the small intestine when required, this serves as the rectum and prevents the need for a permanent ileostomy.

As disclosed herein, the system comprises: (a) a computer processing device, optionally connected to a computer network; and (b) a software module executed by the computer processing device to analyze, in a sample, from a subject an amount of autoantibodies that bind two or more isoforms of Glycoprotein 2 (GP2), wherein the system categorizes the autoantibodies into groups, wherein the groups comprise group 1 autoantibodies that bind to isoforms 1 and/or 2 (SEQ ID NO 1 and/or 2) and group 2 autoantibodies that bind to isoforms 3 and/or 4 (SEQ ID NO 3 and/or 4), and the system attributes the sample to a disorder selected from such as Celiac disease (CeD), Crohn's disease (CD) and/or ulcerative colitis (UC), or to a heathy group based on the amount of group 1 or group 2 autoantibodies measured.

Also disclosed herein is a system that comprises:
- two or more isoforms of GP2, wherein at least one of isoforms 1 and/or 2 and at least one of isoforms 3 and/or 4 are present,
- a sample analyzer for determining the amount of autoantibodies in a sample that bind to isoforms of Glycoprotein 2 (GP2), and
- a computer system for preferably automatically receiving and analyzing data obtained, and for correlating the amount of the autoantibodies with a diagnosis of Celiac disease (CeD), Crohn's disease (CD) and/or ulcerative colitis (UC), wherein the amount of autoantibodies is determined in accordance with the method of any one of the preceding claims.

### DETAILED DESCRIPTION OF THE INVENTION

**Table 1: Terminology of GP2 isoforms**

| **Amino acids** | | **Pubmed #** |
|---|---|---|
| 537 | Isoform 1 SEQ ID NO.1 | NP_001007241.2 |
| 534 | Isoform 2 SEQ ID NO.2 | NP_001493.2 |
| 390 | Isoform 3 SEQ ID NO.3 | NP_001007242.2 |
| 387 | Isoform 4 SEQ ID NO.4 | NP_001007243.2 |

**Table 2: Amino Acid sequences of isoforms 1 to 4**

| SEQ ID NO. | Amino Acid Sequence | Description |
|---|---|---|
| SEQ ID NO 1 | | Transcript Variant: This variant (1) represents the longest transcript, although it occurs rarely. It encodes the longest protein (isoform 1). |
| SEQ ID NO 2 | | Transcript Variant: This variant (2) lacks an alternate in-frame segment, compared to variant 1. The resulting protein (isoform 2) is shorter than isoform 1. Isoform 2 is also known as the alpha form. |
| SEQ ID NO 3 | | Transcript Variant: This variant (3) lacks an alternate in-frame segment, compared to variant 1. The resulting protein (isoform 3) has a shorter N-terminus when compared to isoform 1, although the 31 most N-term aas are maintained. |
| | | |
| SEQ ID NO 4 | | Transcript Variant: This variant (4) lacks two alternate in-frame segments, compared to variant 1. The resulting protein (isoform 4) has a shorter N-terminus when compared to isoform 1, although the 31 most N-term aas are maintained. Isoform 4 is also known as the beta form. |

**Table 3: DNA-Sequences (such as cDNA) corresponding to each of the isoforms**

| SEQ ID NO. | Nucleotide Sequence | Description |
|---|---|---|
| SEQ ID NO 5 | | Isoform 1 |
| | | CCDS Database |
| | | CCDS42128.1 |
| | | |
| SEQ ID NO 6 | | Isoform 2 |
| | | CCDS Database |
| | | CCDS10582.2 |
| | | |
| SEQ ID NO 7 | | Isoform 3 |
| | | CCDS Database |
| | | CCDS45433.1 |
| SEQ ID NO 8 | | Isoform 4 |
| | | CCDS Database |
| | | CCDS45432.1 |

The CCDS reference refers to the CCDS project as described in "The consensus coding sequence (CCDS) project: Identifying a common protein-coding gene set for the human and mouse genomes", Pruitt KD, et al, Genome Res. 2009 Jul;19(7):1316-23.

An autoantibody is an antibody (a type of protein) manufactured by the immune system that is directed against one or more of the individual's own proteins. Many autoimmune diseases are associated with and/or caused by such autoantibodies.

The term "autoimmune disease" refers to any given disease associated with and/or caused by the presence of autoantibodies. Autoimmune diseases arise from an abnormal immune response of the body against substances and tissues normally present in the body (autoimmunity). This may be restricted to certain organs or involve a particular tissue.

A preferred autoimmune disease of the invention is inflammatory bowel disease. The term "inflammatory bowel disease" or "IBD" refers to gastrointestinal disorders including, without limitation, Crohn's disease (CD), ulcerative colitis (UC), and indeterminate colitis (IC).

A preferred autoimmune disease of the invention is therefore an autoimmune disease of the digestive or intestinal tract of said subject. Such diseases are characterised in that the autoimmune disorder exhibits autoantibodies that bind components of the digestive or intestinal tract of said subject. Such components of the digestive or intestinal tract may be any organ, tissue, cell or protein found in said area of the subject. The digestive or intestinal tract may be understood as the gastrointestinal tract (GI tract), which refers to the stomach and intestine, and is divided into the upper and lower gastrointestinal tracts, and may include all the structures from the mouth to the anus. The tract may also be divided into foregut, midgut, and hindgut, reflecting the embryological origin of each segment of the tract.

Gastrointestinal (GI)-related autoantibodies (Abs) can be evaluated in autoimmune diseases such as inflammatory bowel disease, autoimmune hepatitis and celiac disease. Such autoantibodies may relate to ANCA (anti-neutrophil cytoplasmic antibodies) and/or ASCA. IgA and IgG ASCA can be detected in sera from patients with Crohn's disease and may be used in order to differentiate Crohn's disease from UC.

The term "sample" includes any biological specimen obtained from an individual. Suitable samples for use in the present invention include, without limitation, whole blood, plasma, serum, saliva, urine, stool, tears, any other bodily fluid, pure pancreatic juices or duodenal juices, tissue samples (e.g., biopsy) and cellular extracts thereof (e.g., red blood cellular extract). In a preferred embodiment, the sample is a serum sample. The use of samples such as serum, saliva, and urine is well known in the art (see, e.g., Hashida et al., J. Clin. Lab. Anal., 11:267-86 (1997)). One skilled in the art will appreciate that samples such as serum samples can be diluted prior to analysis.

The term "individual," "subject," or "patient" typically refers to humans, but also to other animals including, e.g., other primates, rodents, canines, felines, equines, ovines, porcines, and the like.

As used herein, the term "substantially the same amino acid sequence" includes an amino acid sequence that is similar, but not identical to, the naturally-occurring amino acid sequence. For example, an amino acid sequence, i.e., polypeptide, that has substantially the same amino acid sequence as the GP2 isoforms in SEQ ID NO 1 to 4 and can have one or more modifications such as amino acid additions, deletions, or substitutions relative to the amino acid sequence of the GP2 isoforms, provided that the modified polypeptide retains substantially at least one biological activity of GP2 such as immunoreactivity, in particular the immune reactivity specific to the diseases capable of being diagnosed according to the present invention. A particularly useful modification of a polypeptide of the present invention, or a fragment thereof, is a modification that confers, for example, increased stability or reactivity. Incorporation of one or more D-amino acids is a modification useful in increasing stability of a polypeptide or polypeptide fragment. Similarly, deletion or substitution of lysine residues can increase stability by protecting the polypeptide or polypeptide fragment against degradation.

As used herein, the term "GP2 isoform" includes a protein that has at least about 50% amino acid identity with one or more SEQ ID No 1 to 4. As a non-limiting example, an GP2 isoform of the invention can have at least about 60%, 65%, 70%, 75%, 80%, 85%, 90%, 91%, 92%, 93%, 94%, 95%, 96%, 97%, 98%, or 99% amino acid sequence identity with one or more SEQ ID No 1 to 4. Nucleic acid variants to SEQ ID NO 5 to 8 are also encompassed herein that encode a protein sequence of SEQ ID NO 1 to 4, or a sequence with substantially the same amino acid sequence. The complementary nucleic acid sequence is also encompassed, as is a degenerate sequence modified to use the degenerate nature of the genetic code, as is known to a skilled person.

The amino acid sequences may also comprise 0 to 100, 2 to 50, 5 to 20, or for example 8 to 15, or any value from 0 to 20, amino acid additions or deletions at either the N- and/or C-terminus of the proteins. The termini may also be modified with additional linker sequences, or removal of sequences, as long as the autoantibody binding properties and immunoreactivity of the protein is essentially maintained and the autoantibodies as described herein bind in an analogous manner to the specific sequence provided.

Various ways of preparing functionally analogous peptides have been disclosed in the prior art. Peptides designed starting from the peptides of the invention using such methods are included in the teaching according to the invention. For example, one way of generating functionally analogous peptides has been described in PNAS USA 1998, Oct. 13, 9521, 12179-84; WO 99/6293 and/or WO 02/38592; the above teachings are hereby incorporated in the disclosure of the invention. That is, all peptides, peptide fragments or structures comprising peptides generated using the methods mentioned above - starting from the peptides of the invention - are peptides according to the invention, provided they accomplish the object of the invention and, in particular, interact with the pathogenic autoantibodies. For example, these autoantibodies can be agonistic autoantibodies activating receptors.

The GP2 isoforms may also be described as antigens, as they react with an antibody targeted to said GP2 isoform protein. The GP2 isoforms may also be referred to as proteins or targets. For use in the methods of the invention, a GP2 antigen can be partially purified. A GP2 antigen also can be prepared recombinantly by expressing an encoding nucleic acid sequence as described herein using methods well known in the art (see, for example, Ausubel et al., Current Protocols in Molecular Biology John Wiley & Sons, Inc. New York (1999)).

The term "diagnosing" includes the use of the devices, methods, and systems, of the present invention to determine the presence or absence or likelihood of presence or absence of a medically relevant disorder in an individual. The term also includes devices, methods, and systems for assessing the level of disease activity in an individual. In some embodiments, statistical algorithms are used to diagnose a mild, moderate, severe, or fulminant form of the disorder based upon the criteria developed by Truelove et al., Br. Med. J., 12:1041-1048 (1955). In other embodiments, statistical algorithms are used to diagnose a mild to moderate, moderate to severe, or severe to fulminant form of the IBD based upon the criteria developed by Hanauer et al., Am. J. Gastroenterol., 92:559-566 (1997). As disclosed herein, the presence of GP2 antibodies is used to diagnose Crohn's disease. One skilled in the art will know of other methods for evaluating the severity of IBD in an individual.

The comparative analysis described herein between autoantibody binding to different GP2 isoforms is a preferred method of the present invention. Direct comparison based on autoantibody binding as measured in the same experiment may be used. For this embodiment the amount of GP2 isoform provided for the experiment should be controlled carefully to enable direct comparative analysis. Alternatively, or in combination, control values or standards may be used that provide samples with autoantibodies or represent control amounts thereof, as have already been obtained from previous analytical tests. It is possible to use control values having been generated by the testing of cohorts or other large numbers of subjects suffering from any given disease or control group. Appropriate statistical means are known to those skilled in the art for analysis and comparison of such data sets. Control samples for positive controls (such as disease sufferers) or negative controls (from healthy subjects) may be used for reference values in either simultaneous of non-simultaneous comparison.

The invention also encompasses use of the method for disease monitoring, also known as monitoring the progression or regression of the autoimmune disease. The term "monitoring the progression or regression of the autoimmune disease" includes the use of the devices, methods, and systems of the present invention to determine the disease state (e.g., presence or severity of the autoimmune disease) of an individual. In certain instances, the results of a statistical algorithm (e.g., a learning statistical classifier system) are compared to those results obtained for the same individual at an earlier time. In some aspects, the devices, methods, and systems of the present invention can also be used to predict the progression of the autoimmune disease, e.g., by determining a likelihood for the autoimmune disease to progress either rapidly or slowly in an individual based on the presence or level of at least one marker in a sample. In other aspects, the devices, methods, and systems of the present invention can also be used to predict the regression of the autoimmune disease, e.g., by determining a likelihood for the autoimmune disease to regress either rapidly or slowly in an individual based on the presence or level of at least one marker in a sample. Therapy monitoring may also be conducted, whereby a subject is monitored for disease progression during the course of any given therapy.

In certain instances, the presence or level of anti-GP2 antibodies or at least one marker is determined using an immunoassay or an immunohistochemical assay. A non-limiting example of an immunoassay suitable for use in the method of the present invention includes an ELISA. Examples of immunohistochemical assays suitable for use in the method of the present invention include, but are not limited to, immunofluorescence assays such as direct fluorescent antibody assays, IFA assays, anticomplement immunofluorescence assays, and avidin-biotin immunofluorescence assays. Other types of immunohistochemical assays include immunoperoxidase assays.

Celiac disease (CeD) is an autoimmune disorder of the small intestine that occurs in people of all ages from infancy onward. Symptoms include discomfort in the digestive tract, chronic constipation and diarrhoea, anaemia and fatigue, but these may be absent, and symptoms in other organ systems have been described. Severe CeD leads to the characteristic symptoms of pale, loose and greasy stool (steatorrhoea) and weight loss or failure to gain weight (in young children). People with milder coeliac disease may have symptoms that are much more subtle and occur in other organs than the bowel itself. It is also possible to have coeliac disease without any symptoms whatsoever. Abdominal pain and cramping, bloatedness with abdominal distension and mouth ulcers may be present. As the bowel becomes more damaged, a degree of lactose intolerance may develop. Frequently, the symptoms are ascribed to irritable bowel syndrome (IBS), only later to be recognised as coeliac disease; a small proportion of people with symptoms of IBS have underlying coeliac disease, and screening for coeliac disease is recommended for those with IBS symptoms.

Crohn's disease (CD) is a disease of chronic inflammation that can involve any part of the gastrointestinal tract. Commonly, the distal portion of the small intestine, i.e., the ileum, and the cecum are affected. In other cases, the disease is confined to the small intestine, colon, or anorectal region. CD occasionally involves the duodenum and stomach, and more rarely the esophagus and oral cavity. The variable clinical manifestations of CD are, in part, a result of the varying anatomic localization of the disease. The most frequent symptoms of CD are abdominal pain, diarrhea, and recurrent fever. CD is commonly associated with intestinal obstruction or fistula, an abnormal passage between diseased loops of bowel.

Crohn's disease belongs to the group of chronic inflammatory bowel diseases. It is a presumably autoaggressive, chronic-granulomatous inflammation which may appear in the entire gastrointestinal tract, i.e. from the oral cavity down to the anus. Affection is mainly in the lower small intestine (terminal ileum, affection about 40%) and colon, more rarely in esophagus and mouth. Crohn's disease is characterized by a discontinuous, segmental affection (so-called "skip lesions") of the intestinal mucosa, i.e., the disease can be present simultaneously in a plurality of intestinal sections separated by healthy sections. Other designations of the disease are regional enteritis, terminal ileitis, regional enterocolitis and sclerosing chronic enteritis, or the abbreviation CD (Crohn's disease), and the autoimmune disease (inflammatory bowel disease) as a generic term. Accordingly, Crohn's disease in the meaning of the invention is any condition which is macroscopically characterized by the following changes: Garden hose phenomenon: segmental stenoses caused by fibrosing, Cobble stone phenomenon: inflamed mucosa in alternation with deep ulcerations, thereby producing a cobble stone-like appearance, or Inflammatory conglomerate tumor: various intestinal sections adhere to each other.

Ulcerative colitis (UC) is a disease of the large intestine characterized by chronic diarrhea with cramping, abdominal pain, rectal bleeding, loose discharges of blood, pus, and mucus. The manifestations of UC vary widely. A pattern of exacerbations and remissions typifies the clinical course for about 70% of UC patients, although continuous symptoms without remission are present in some patients with UC. Local and systemic complications of UC include arthritis, eye inflammation such as uveitis, skin ulcers, and liver disease. In addition, UC, and especially the long-standing, extensive form of the disease is associated with an increased risk of colon carcinoma.

Pancreatitis in the meaning of the invention is inflammation of the pancreas which can be acute or take a chronic course. Pancreatitis is usually induced by activation of pancreatic enzymes within the organ. The function of these enzymes is to digest proteins and fat so that autodigestion of the organ is induced. Autodigestion results in inflammation of the pancreas. In severe cases, hemorrhage, serious tissue damage, infections and cysts may develop. An inflamed gland may cause enzymes to enter the bloodstream, thus reaching the lungs, heart and kidneys where further damage may arise. Acute pancreatitis develops when the pancreas suddenly becomes inflamed but recovers afterwards. Some patients suffer from acute pancreatitis a number of times but recover completely each time. Acute pancreatitis appears suddenly and can be a serious, life-threatening disease causing a large number of complications, but the patients normally recover from acute pancreatitis. The incidence is about five to ten new diseases per 100,000 inhabitants per year.

According to the invention, the GP2 isoforms described herein are used to detect primary sclerosing cholangitis. Also disclosed herein is the use of the GP2 isoforms described herein to detect hepatic diseases or autoimmune enteritides. Most surprisingly, the GP2 autoantigen is suitable not only for specific detection of the autoimmune disease, but also for the detection of various hepatic diseases.

Cholangitis in the meaning of the invention refers to inflammation of the intrahepatic biliary ducts. It can be induced by various causes, including - among other things - obstruction of the biliary ducts by gallstones, stenoses, tumors or parasite infestation. It is differentiated into acute purulent cholangitis, non-purulent destructive cholangitis and chronic sclerosing cholangitis.

Autoimmune enteritides in the meaning of the invention involve any form of enteritis, especially those being caused by chronic inflammatory bowel diseases. Also, autoimmune enteritides in the meaning of the invention involve those being caused by salmonella, E. coli, cholera or typhus pathogens, or by fungi, protozoa, toxic substances, but also any allergy-based enteritis or any form of actinic enteritis, Yersinia enteritis or bacterial dysentery.

As used herein, the term "antibody" includes a population of immunoglobulin molecules, which can be polyclonal or monoclonal and of any isotype, or an immunologically active fragment of an immunoglobulin molecule. Such an immunologically active fragment contains the heavy and light chain variable regions, which make up the portion of the antibody molecule that specifically binds an antigen. For example, an immunologically active fragment of an immunoglobulin molecule known in the art as Fab, Fab' or F(ab')2 is included within the meaning of the term antibody.

In another advantageous embodiment the immunoassay is used in the detection of antibodies, to which end binding of the GP2 isoform antigen to a solid phase is envisaged. Following addition of sample solution, the patient's antibody included therein binds to the GP2 antigen. The antibody which is obtained e.g. from the serum or stool of a patient and bound to GP2 is subsequently detected using a label, or labelled reagent and optionally quantified. Thus, according to the invention, detection of the antibodies in this method is effected using labelled reagents according to the well-known ELISA (Enzyme-Linked Immunosorbent Assay) technology. Labels according to the invention therefore comprise enzymes catalyzing a chemical reaction which can be determined by optical means, especially by means of chromogenic substrates, chemiluminescent methods or fluorescent dyes. In another preferred embodiment the autoantibodies are detected by labelling with weakly radioactive substances in radioimmunoassays (RIA) wherein the resulting radioactivity is measured.

As examples of means for detecting the label in the method of the present invention, a variety of immunoassay techniques, including competitive and non-competitive immunoassays, can be used to determine the presence or level of one or more markers in a sample (see, e.g., Self et al., Curr. Opin. Biotechnol., 7:60-65 (1996)). The term immunoassay encompasses techniques including, without limitation, enzyme immunoassays (EIA) such as enzyme multiplied immunoassay technique (EMIT), enzyme-linked immunosorbent assay (ELISA), antigen capture ELISA, sandwich ELISA, IgM antibody capture ELISA (MAC ELISA), and microparticle enzyme immunoassay (MEIA); capillary electrophoresis immunoassays (CEIA); radioimmunoassays (RIA); immunoradiometric assays (IRMA); fluorescence polarization immunoassays (FPIA); and chemiluminescence assays (CL). If desired, such immunoassays can be automated. Immunoassays can also be used in conjunction with laser induced fluorescence (see, e.g., Schmalzing et al., Electrophoresis, 18:2184-2193 (1997); Bao, J. Chromatogr. B. Biomed. Sci., 699:463-480 (1997)). Liposome immunoassays, such as flow-injection liposome immunoassays and liposome immunosensors, are also suitable for use in the present invention (see, e.g., Rongen et al., J. Immunol. Methods, 204:105-133 (1997)). In addition, nephelometry assays, in which the formation of protein/antibody complexes results in increased light scatter that is converted to a peak rate signal as a function of the marker concentration, are suitable for use in the present invention. Nephelometry assays are commercially available from Beckman Coulter (Brea, Calif.; Kit #449430) and can be performed using a Behring Nephelometer Analyzer (Fink et al., J. Clin. Chem. Clin. Biol. Chem., 27:261-276 (1989)).

The immunoassays described above are particularly useful for determining the presence or level of one or more markers in a sample (and may be considered examples of means for detecting a label), wherein a marker may be understood as an autoantibody targeted to an isoform of GP2. As a non-limiting example, a fixed neutrophil ELISA is useful for determining whether a sample is positive for ANCA or for determining ANCA levels in a sample. Similarly, an ELISA using yeast cell wall phosphopeptidomannan is useful for determining whether a sample is positive for ASCA-IgA and/or ASCA-IgG, or for determining ASCA-IgA and/or ASCA-IgG levels in a sample. An ELISA using GP2 isoform protein or a fragment thereof is useful for determining whether a sample is positive for anti-GP2 antibodies, or for determining anti-GP2 antibody levels in a sample.

In another preferred embodiment of the method according to the invention the autoantibodies are detected in an immunoassay, preferably with direct or indirect coupling of one reactant to a labelling substance. This enables flexible adaptation of the method to the potentials and requirements of different laboratories and their laboratory diagnostic equipment. In one advantageous embodiment the the autoimmune disease-specific antibodies are detected in an immunoassay wherein the antibodies are present dissolved in a liquid phase, preferably diluted in a conventional buffer solution well-known to those skilled in the art or in an undiluted body fluid. According to the invention, detection can also be effected using stool samples.

In another preferred embodiment of the invention, soluble or solid phase-bound GP2 molecules are used to bind the antibodies. In a second reaction step, anti-human immunoglobulins are employed, preferably selected from the group comprising anti-human IgA, anti-human IgM and/or anti-human IgG antibodies, said anti-human immunoglobulins being detectably labelled conjugates of two components which can be conjugated with any conventional labelling enzymes, especially chromogenic and/or chemiluminescent substrates, preferably with horseradish peroxidase, alkaline phosphatase. The advantage of this embodiment lies in the use of ELISA technology usually available in laboratory facilities so that detection according to the invention can be established in a cost-effective manner. In another preferred embodiment of the invention the antibody bound to GP2 reacts with anti-human immunoglobulins, preferably selected from the group comprising anti-human IgA, anti-human IgM and/or anti-human IgG antibodies, detectably coupled to fluorescein isothiocyanate (FITC). Much like the above-mentioned ELISA, the FITC technology represents a system that is available in many places and therefore allows smooth and low-cost establishment of the inventive detection in laboratory routine.

Specific immunological binding of the antibody to the marker of interest can be detected directly or indirectly via a label. Any given means for detecting these labels may be considered means for detecting the label according to the method of the invention. Direct labels include fluorescent or luminescent tags, metals, dyes, radionuclides, and the like, attached to the antibody. An antibody labeled with iodine-125 (1251) can be used for determining the levels of one or more markers in a sample. A chemiluminescence assay using a chemiluminescent antibody specific for the marker is suitable for sensitive, non-radioactive detection of marker levels. An antibody labeled with fluorochrome is also suitable for determining the levels of one or more markers in a sample. Examples of fluorochromes include, without limitation, DAPI, fluorescein, Hoechst 33258, R-phycocyanin, B-phycoerythrin, R-phycoerythrin, rhodamine, Texas red, and lissamine. Secondary antibodies linked to fluorochromes can be obtained commercially, e.g., goat F(ab')2 anti-human IgG-FITC is available from Tago Immunologicals (Burlingame, Calif.).

Indirect labels include various enzymes well-known in the art, such as horseradish peroxidase (HRP), alkaline phosphatase (AP), β-galactosidase, urease, and the like. A horseradish-peroxidase detection system can be used, for example, with the chromogenic substrate tetramethylbenzidine (TMB), which yields a soluble product in the presence of hydrogen peroxide that is detectable at 450 nm. An alkaline phosphatase detection system can be used with the chromogenic substrate p-nitrophenyl phosphate, for example, which yields a soluble product readily detectable at 405 nm. Similarly, a β-galactosidase detection system can be used with the chromogenic substrate o-nitrophenyl-β-D-galactopyranoside (ONPG), which yields a soluble product detectable at 410 nm.

A signal from the direct or indirect label can be analyzed, for example, using a spectrophotometer to detect colour from a chromogenic substrate; a radiation counter to detect radiation such as a gamma counter for detection of 1251; or a fluorometer to detect fluorescence in the presence of light of a certain wavelength. For detection of enzyme-linked antibodies, a quantitative analysis of the amount of marker levels can be made using a spectrophotometer such as an EMAX Microplate Reader (Molecular Devices; Menlo Park, Calif.) in accordance with the manufacturer's instructions. If desired, the assays of the present invention can be automated or performed robotically, and the signal from multiple samples can be detected simultaneously.

In certain embodiments, the present invention provides methods of diagnosing the autoimmune disease or clinical subtypes thereof using GP2 isoforms. A variety of inflammatory bowel disease (the autoimmune disease) markers, such as biochemical markers, serological markers, genetic markers, or other clinical or echographic characteristics, are suitable for use and can be combined with statistical algorithms to classify a sample from an individual as an the autoimmune disease sample. Examples of markers of the diseases (an autoantibody directed against the GP2 isoforms described herein) suitable for use in the present invention include, but are not limited to, anti-neutrophil antibodies (e.g., ANCA, pANCA, cANCA, NSNA, SAPPA, etc.) or anti-Saccharomyces cerevisiae antibodies (e.g., ASCA-IgA, ASCA-IgG, ASCA-IgM, etc.).One skilled in the art will know of additional markers suitable for use in the statistical algorithms of the present invention.

The determination of ANCA levels and/or the presence or absence of pANCA in a sample is useful in the present invention. As used herein, the term "anti-neutrophil cytoplasmic antibody" or "ANCA" includes antibodies directed to cytoplasmic and/or nuclear components of neutrophils. ANCA activity can be divided into several broad categories based upon the ANCA staining pattern in neutrophils: (1) cytoplasmic neutrophil staining without perinuclear highlighting (cANCA); (2) perinuclear staining around the outside edge of the nucleus (pANCA); (3) perinuclear staining around the inside edge of the nucleus (NSNA); and (4) diffuse staining with speckling across the entire neutrophil (SAPPA). ANCA levels in a sample from an individual can be determined, for example, using an immunoassay such as an enzyme-linked immunosorbent assay (ELISA) with alcohol-fixed neutrophils.

The determination of ASCA (e.g., ASCA-IgA and/or ASCA-IgG) levels in a sample is also useful in the present invention. As used herein, the term "anti-Saccharomyces cerevisiae immunoglobulin A" or "ASCA-IgA" includes antibodies of the immunoglobulin A isotype that react specifically with S. cerevisiae. Similarly, the term "anti-Saccharomyces cerevisiae immunoglobulin G" or "ASCA-IgG" includes antibodies of the immunoglobulin G isotype that react specifically with S. cerevisiae. The determination of whether a sample is positive for ASCA-IgA or ASCA-IgG is made using an antigen specific for ASCA. Such an antigen can be any antigen or mixture of antigens that is bound specifically by ASCA-IgA and/or ASCA-IgG. Although ASCA antibodies were initially characterized by their ability to bind S. cerevisiae, those of skill in the art will understand that an antigen that is bound specifically by ASCA can be obtained from S. cerevisiae or from a variety of other sources as long as the antigen is capable of binding specifically to ASCA antibodies.

The invention also relates to protein and nucleic acid molecules corresponding to the sequences described herein, for example proteins or nucleic acid molecules comprising or consisting of said sequences.

The determination of autoantibodies to the novel GP2-isoforms or use thereof in an ELISA as a solid-phase antigen in serological diagnostics of the diseases described herein has neither been considered nor mentioned in the prior art.

In another aspect, the invention relates to a method wherein human IgA, IgM and/or IgG antibody autoimmune diseases are detected.

As used herein, the term "GP2 isoform", "GP2", "GP2-antigen", "GP2-molecule", "GP2-protein", "GP2-peptide" or "GP2-autoantigen", or other GP2-referencing phrase relates to the novel GP2-isoforms of the sequences as disclosed herein, or functionally analogous sequences thereof, preferably to those isoforms 1, 2, 3 and 4. In a preferred embodiment of the method according to the invention the GP2-isoform is of human, animal, recombinant or synthetic origin. GP2 represents a highly conserved peptide so that GP2 of any origin can advantageously be used for detection as long as the sequence is functionally analog to the sequence according to the invention. High binding affinity between the GP2 as antigen and the autoantibodies is retained.

In another preferred embodiment of the invention the GP2 in accordance with one or more of the sequences disclosed herein is bound to a solid phase. Binding of GP2 in accordance with one or more of the sequences disclosed herein to the solid phase can be effected via a spacer. All those chemical compounds having suitable structural and functional preconditions for spacer function can be used as spacers as long as they do not modify the binding behavior in such a way that binding of the GP2 autoantibody in accordance with one or more of the sequences disclosed herein is adversely affected.

In another preferred embodiment of the invention the molecule comprises a linker or spacer selected from the group of α-aminocarboxylic acids as well as homo- and heterooligomers thereof, α,ω-aminocarboxylic acids and branched homo- or heterooligomers thereof, other amino acids, as well as linear and branched homo- or heterooligomers; amino-oligoalkoxyalkyl-amines; maleinimidocarboxylic acid derivatives; oligomers of alkylamines; 4-alkylphenyl derivatives; 4-oligoalkoxyphenyl or 4-oligoalkoxyphenoxy derivatives; 4-oligoalkylmercaptophenyl or 4-oligoalkylmercaptophenoxy derivatives; 4-oligoalkylaminophenyl or 4-oligoalkylaminophenoxy derivatives; (oligoalkylbenzyl)phenyl or 4-(oligoalkylbenzyl)phenoxy derivatives, as well as 4-(oligoalkoxybenzyl)phenyl or 4-(oligoalkoxybenzyl)phenoxy derivatives; trityl derivatives; benzyloxyaryl or benzyloxyalkyl derivatives; xanthen-3-yloxyalkyl derivatives; (4-alkylphenyl)- or ω-(4-alkylphenoxy)alkanoic acid derivatives; oligoalkylphenoxyalkyl or oligoalkoxyphenoxyalkyl derivatives; carbamate derivatives; amines; trialkylsilyl or dialkylalkoxysilyl derivatives; alkyl or aryl derivatives or combinations thereof.

According to the invention it is also preferred to perform the above-described detection method on a solid phase, for example by connection of the GP2 molecule to the solid phase via a linker, in which case the storability of the peptide is advantageously increased as a result of the surprisingly stable linkage of the GP2 antigen to the solid phase.

In another preferred embodiment of the invention the GP2 molecule is used as a soluble or solid phase-bound autoantigen for direct or indirect autoantibody detection in stool and/or body fluids, especially blood and/or serum, in which case the use of the GP2 molecule in accordance with one or more of the sequences as disclosed herein was found particularly advantageous.

In another preferred embodiment of the invention the sequences according to the present application, or the peptides which can be generated therefrom, are immobilized. More specifically, the solid phase-bound GP2 molecule in accordance with one or more of the sequences as disclosed herein is bound to organic, inorganic, synthetic and/or mixed polymers, preferably agarose, cellulose, silica gel, polyamides and/or polyvinyl alcohols. In the meaning of the invention, immobilization is understood to involve various methods and techniques to fix the peptides on specific carriers, e.g. according to WO 99/56126 or WO 02/26292. For example, immobilization can serve to stabilize the peptides so that their activity would not be reduced or adversely modified by biological, chemical or physical exposure, especially during storage or in single-batch use. Immobilization of the peptides allows repeated use under technical or clinical routine conditions; furthermore, a sample - preferably blood components - can be reacted with at least one of the peptides according to the invention in a continuous fashion. In particular, this can be achieved by means of various immobilization techniques, with binding of the peptides to other peptides or molecules or to a carrier proceeding in such a way that the three-dimensional structure - particularly in the active center mediating the interaction with the autoantibodies - of the corresponding molecules, especially of said peptides, would not be changed. Advantageously, there is no loss in specificity to the autoantibodies of patients as a result of such immobilization. In the meaning of the invention, three basic methods can be used for immobilization:
(i) Crosslinking: in crosslinking, the peptides are fixed to one another without adversely affecting their activity. Advantageously, they are no longer soluble as a result of such crosslinking.
(ii) Binding to a carrier: binding to a carrier proceeds via adsorption, ionic binding or covalent binding, for example. Such binding may also take place inside microbial cells or liposomes or other membranous, closed or open structures. Advantageously, the peptides are not adversely affected by such fixing. For example, multiple or continuous use of carrier-bound peptides is possible with advantage in clinical diagnosis or therapy.
(iii) Inclusion: inclusion in the meaning of the invention especially proceeds in a semipermeable membrane in the form of gels, fibrils or fibers. Advantageously, encapsulated peptides are separated from the surrounding sample solution by a semipermeable membrane in such a way that interaction with the autoantibodies or fragments thereof still is possible. Various methods are available for immobilization, such as adsorption on an inert or electrically charged inorganic or organic carrier. For example, such carriers can be porous gels, aluminum oxide, bentonite, agarose, starch, nylon or polyacrylamide. Immobilization proceeds via physical binding forces, frequently involving hydrophobic interactions and ionic binding. Advantageously, such methods are easy to handle and have little influence on the conformation of the peptides. Advantageously, binding can be improved as a result of electrostatic binding forces between the charged groups of the peptides and the carrier, e.g. by using ion exchangers, particularly Sephadex.

Another method is covalent binding to carrier materials. In addition, the carriers may have reactive groups forming homopolar bonds with amino acid side chains. Suitable groups in peptides are carboxy, hydroxy and sulfide groups and especially the terminal amino groups of lysines. Aromatic groups offer the possibility of diazo coupling. The surface of microscopic porous glass particles can be activated by treatment with silanes and subsequently reacted with peptides. For example, hydroxy groups of natural polymers can be activated with bromocyanogen and subsequently coupled with peptides. Advantageously, a large number of peptides can undergo direct covalent binding with polyacrylamide resins. Inclusion in three-dimensional networks involves inclusion of the peptides in ionotropic gels or other structures well-known to those skilled in the art. More specifically, the pores of the matrix are such in nature that the peptides are retained, allowing interaction with the target molecules. In crosslinking, the peptides are converted into polymer aggregates by crosslinking with bifunctional agents. Such structures are gelatinous, easily deformable and, in particular, suitable for use in various reactors. By adding other inactive components such as gelatin in crosslinking, advantageous improvement of mechanical and binding properties is possible. In microencapsulation, the reaction volume of the peptides is restricted by means of membranes. For example, microencapsulation can be carried out in the form of an interfacial polymerization. Owing to the immobilization during microencapsulation, the peptides are made insoluble and thus reusable. In the meaning of the invention, immobilized peptides are all those peptides being in a condition that allows reuse thereof. Restricting the mobility and solubility of the peptides by chemical, biological or physical means advantageously results in lower process cost, particularly when eliminating autoantibodies from blood components.

The invention also relates to a diagnostic kit for the determination of autoimmune diseases, comprising a GP2 isoform molecule in accordance with one or more of the sequences as disclosed herein. The diagnostic kit optionally includes instructions concerning combining the contents of the kit and/or providing a formulation for the detection of inflammatory bowel diseases, particularly Crohn's disease, chronic pancreatitis, celiac disease and/or ulcerative colitis. For example, the instruction can be in the form of an instruction leaflet or other medium providing the user with information as to the type of method wherein the substances mentioned are to be used. Obviously, the information need not necessarily be in the form of an instruction leaflet, and the information may also be imparted via the Internet, for example. To a patient, one advantageous effect of such a kit is, for instance, that he or she, without directly addressing a physician, can determine the actual state of a disease even during a journey and optionally adapt diet and activities accordingly.

### FIGURES

Without intending to be limiting, the invention will be explained in more detail with reference to the figures.
Figures of Example 1:

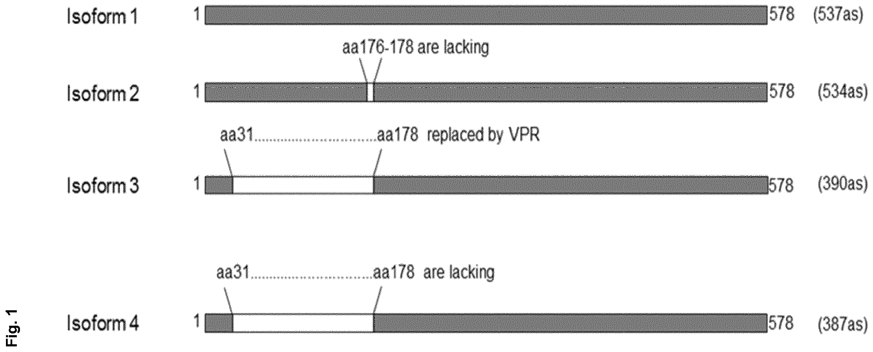

   Fig. 1: Amino acid (AA) differences of the 4 isoforms of glycoprotein 2 (V, valine; P, proline; R, arginine).

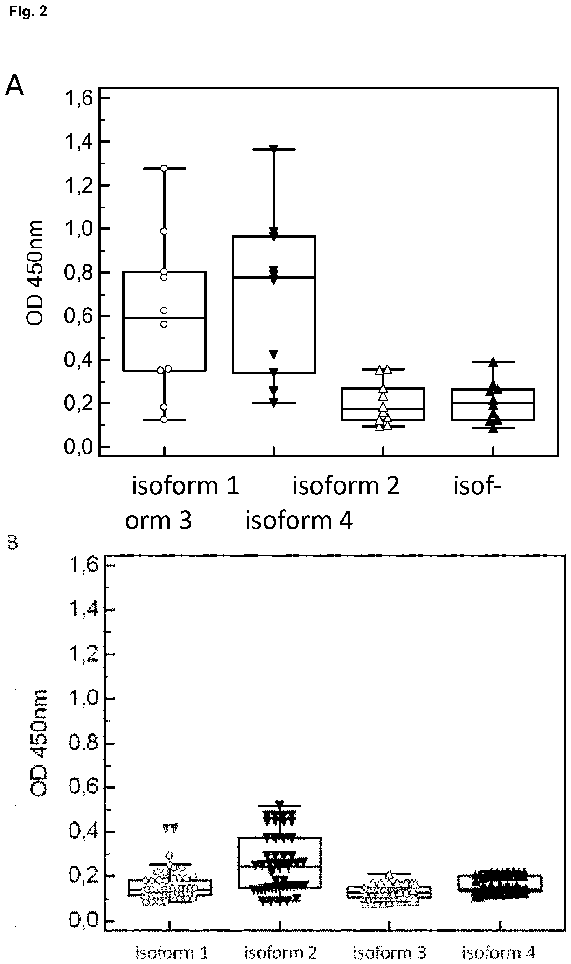

   Fig. 2: Reactivity of IgG to 4 different GP2 isoforms by ELISA in 10 patients with de-novo celiac disease (A) and 50 blood donors (B). Data are displayed as optical densities (OD) in Box-and-Whisker plots with far out values, defined as values that are smaller than the lower quartile minus 3 times the interquartile range, or larger than the upper quartile plus 3 times the interquartile range, displayed as triangles.

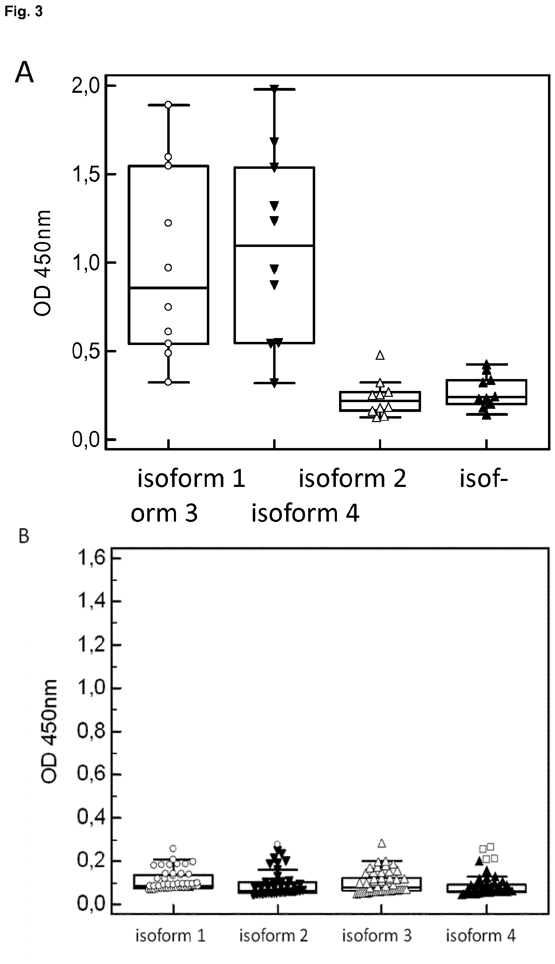

   Fig. 3: Reactivity of IgA to 4 different GP2 isoforms by ELISA in 10 patients with de-novo celiac disease (A) and 50 blood donors (B). Data are displayed as optical densities (OD) in Box-and-Whisker plots with far out values, defined as values that are smaller than the lower quartile minus 3 times the interquartile range, or larger than the upper quartile plus 3 times the interquartile range, displayed as rectangles.

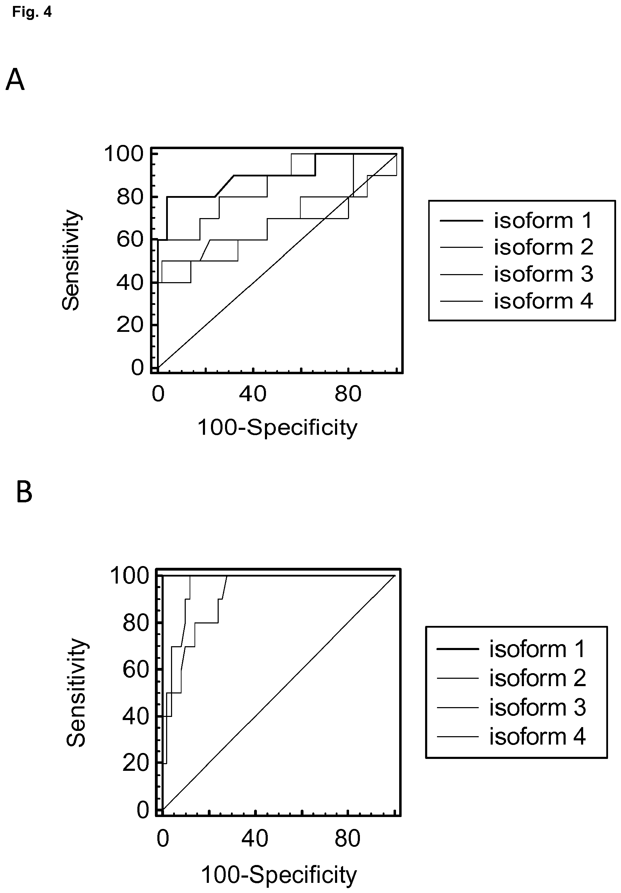

   Fig. 4: Receiver operating characteristics curve analysis of IgG (A) and IgA (B) to 4 different GP2 isoforms by ELISA in 10 patients with de-novo celiac disease (A) and 50 blood donors (B)
Figures of Example 2:

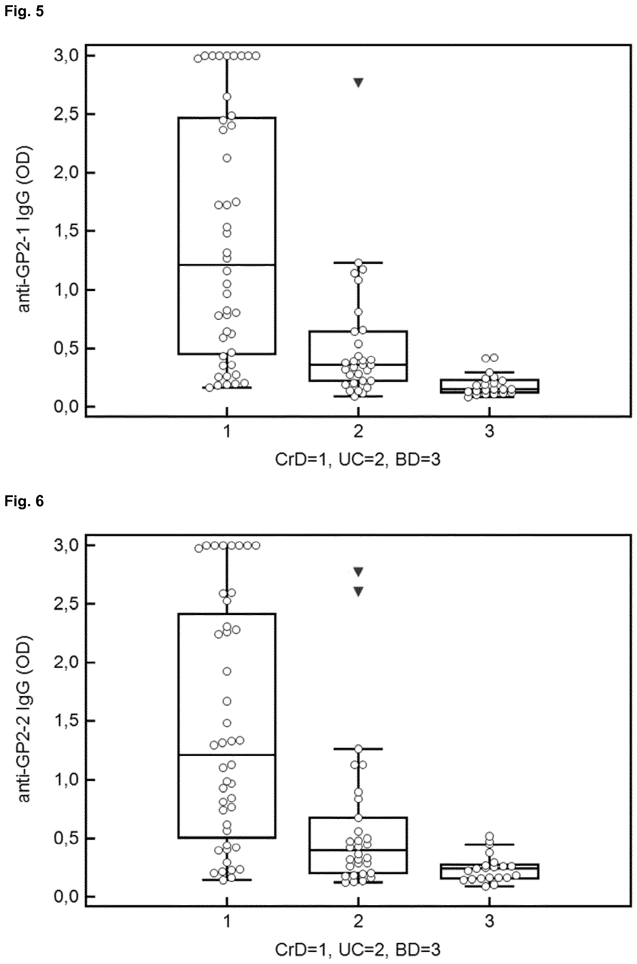

   Fig. 5: Reactivitiy of IgG to Isoform 1 of GP2 in 44 patients with CD, 30 patients with CU and 21 blood donors. Data are displayed as optical densities (OD) in Box-and-Whisker plots with far out values, defined as values that are smaller than the lower quartile minus 3 times the interquartile range, or larger than the upper quartile plus 3 times the interquartile range, displayed as triangles.
   Fig. 6: Reactivitiy of IgG to Isoform 2 of GP2 in 44 patients with CD, 30 patients with CU and 21 blood donors. Data are displayed as optical densities (OD) in Box-and-Whisker plots with far out values, defined as values that are smaller than the lower quartile minus 3 times the interquartile range, or larger than the upper quartile plus 3 times the interquartile range, displayed as triangles.

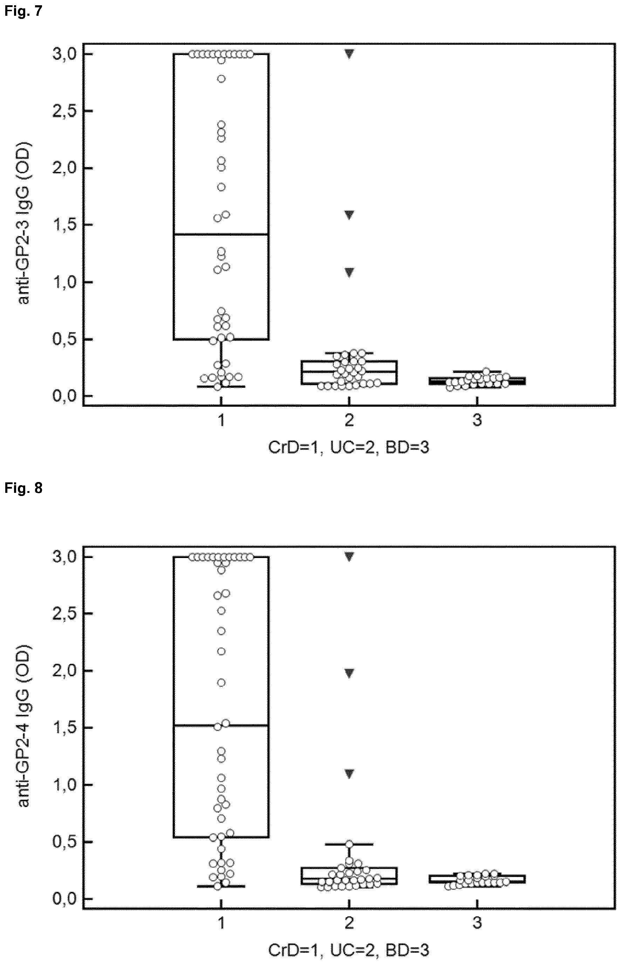

   Fig. 7: Reactivitiy of IgG to Isoform 3 of GP2 in 44 patients with CD, 30 patients with CU and 21 blood donors. Data are displayed as optical densities (OD) in Box-and-Whisker plots with far out values, defined as values that are smaller than the lower quartile minus 3 times the interquartile range, or larger than the upper quartile plus 3 times the interquartile range, displayed as triangles.
   Fig. 8: Reactivitiy of IgG to Isoform 4 of GP2 in 44 patients with CD, 30 patients with CU and 21 blood donors. Data are displayed as optical densities (OD) in Box-and-Whisker plots with far out values, defined as values that are smaller than the lower quartile minus 3 times the interquartile range, or larger than the upper quartile plus 3 times the interquartile range, displayed as triangles.
References in the figures to CrD refer to CD.

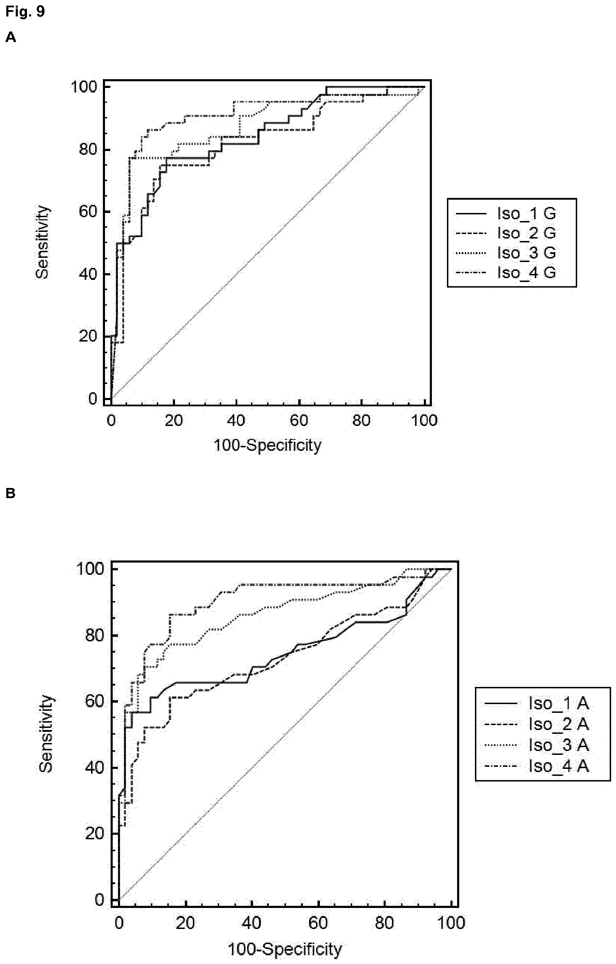

   Fig. 9: Receiver operating characteristics curve analysis of IgG (A) and IgA (B) to 4 different GP2 isoforms in 44 patients with CD, 30 patients with CU and 21 blood donors.

### EXAMPLES

Without intending to be limiting, the invention will be explained in more detail with reference to an example.

The experiments provede herein demonstrate that autoantibodies targeted to the longer isoforms of GP2 (1 and 2) can be used for the diagnosis of de-novo celiac disease and are characterized by better assay performance characteristics than autoantibodies in comparison to the shorter isoforms. Autoantibodies to the shorter isoforms of GP2, in particular isoform 4, can be used for the differential diagnosis of inflammatory bowel diseases and show a better differentiation of Crohn's disease and ulcerative colitis patients.

### Materials:

### Expression of GP2 in Spodoptera frugiperda 9 cells

For expression of the four GP2 isoforms, four plasmids coding the amino acid sequence of the GP2 isoforms NP_001007241.2 (isoform 1), NP_001493.2 (isoform 2), NP_001007242.2 (isoform 3) and NP_001007243.2 (isoform 4) (fig. 1, tables 1-3) were employed. A thrombin cleaving site (VPRGS) and a His6-Tag were added at the C-terminal end. The obtained insert was ligated into a BamHI and EcoRI site of a pVL1393-vector resulting into 4 plasmids (pVL-GP2xtrunc-Thrombin-His) and the constructs were verified by sequencing.

For transfection, 106 Sf9 cells were disseminated in culture medium TC-100 (Biochrom, Berlin, Germany) containing 10% FCS, 6 mM glutamine, 50 µg/ml streptomycin, and 50 U/ml penicillin, and incubated in a 25 cm2 flask for at least two hours at 28OC. For transfection, two solutions were prepared containing 2.5 µg BaculoGold (Virus-DNA, BD Biosciences, San Jose, CA) and 2.5 µg DNA of the pVL-GP2xtrunc-Thrombin-His vectors and Polyfect transfection reagent (Qiagen, Hilden, Germany) dissolved in distilled water, respectively. Both solutions were mixed and incubated at room temperature (RT) for 20 minutes.

The Sf9 culture medium was replaced with serum-free TC-100 and the prepared transfection solution was added to the Sf9 culture and incubated overnight at 28OC, subsequently. Afterwards, the medium was exchanged for culture medium. After 5 days, supernatant containing the recombinant virus was collected and used for further infections.

For infection of Sf9 cells with the GP2-DNA containing Baculovirus, Sf9 cells were disseminated into 150 cm² flasks with a density of 2x106 cells/cm2. The supernatant of a previous infected culture was added at a ratio of 1 to 10 and cultures were incubated on a shaker (100 rpm) at 28OC for 3 days. After harvesting the supernatant for purification of the secreted GP2 isoform, GP2 expression was controlled by sodium dodecyl sulphate polyacrylamid gelelectrophoresis (SDS-PAGE) and Western blot using anti-His and anti-human GP2 antibodies as described elsewhere.

### Purification of GP2 isoforms by Ni-chelate and ion exchange chromatography

Harvested supernatants were clarified by centrifugation and subjected to an ÄKTA-FPLC (GE Healthcare, München, Germany) controlled Ni-chelate column (HisTrap, 1 ml, GE Healthcare) equilibrated with binding buffer (20 mM Na-phosphate, 200 mM NaCl, 50 mM imidazol, pH8.0). To remove unbound protein, the column was subsequently rinsed with 7 ml binding buffer. Bound proteins were eluted with 500 mM imidazol in a 20 mM Na-phosphate solution (pH7.5). Fractions were collected and stored with 0.01% sodium azide at 4OC until further purification.

For anion exchange chromatography, a Mono Q 5/50 column (SGE Healthcare) was equilibrated with 20 mM Na-phosphate (pH7.5). After application of the GP2 containing solution, bound proteins were eluted with a 1 M NaCl gradient in a 20 mM Na-phosphate buffer (pH7.5). Glycoprotein 2 containing fractions were pooled, dialyzed against 50 mM Tris buffer (pH7.5), and stored with 0.01% sodium azide at 4OC until further use.

### Detection of anti-GP2 isoform antibodies by ELISA

IgG and IgA against the 4 GP2 isoforms were assessed in serum samples of patients and controls by an enzyme-linked immunosorbent assay (ELISA). As solid-phase antigenic targets, these assays employ the different recombinant human GP2 isoforms expressed in *Spodoptera frugiperda* 9 cells. Briefly, GP2 isoforms at a concentration of 5 µg/ml were coated onto Maxisorb microtiter plates (Nunc, Roskilde, Denmark) in coating buffer (pH 9.5) at 4°C. After blocking at RT for one hour, serum samples diluted 1 in 100 were incubated at RT for one hour and washed. Horseradish peroxidase-conjugated anti-human IgG or IgA antibodies (Seramun GmbH, Heidesee, Germany) were added and developed with ready-to-use hydrogen peroxide / tetramethylbenzidine substrate (Seramun, Heidesee, Germany). The reaction was stopped with 0.25 mol/l sulphuric acid after 15 minutes. The optical density (OD) of the samples was read using a microplate reader (SLT, Crailsheim, Germany) at a wavelength of 450 nm / 620 nm.

### ELISA for the detection of CeD-specific antibodies

Serum IgA and IgG antibodies to human recombinant tTG (anti-tTG) and dDG (anti-dGD) were determined by ELISA according to the instructions of the manufacturer (GA Generic Assays GmbH) [34]. The optical density was read in a microplate reader at 450 nm and results expressed as arbitrary units (U/ml). The cut off for positivity at 10 U/ml in accordance with the recommendations of the manufacturer was used for these assays. The functional assay sensitivity [35] was determined at 2 U/ml and 3 U/ml for anti-tTG and anti-dGD IgA, respectively, and 3 U/ml and 2 U/ml for IgG to tTG and dGD, respectively.

### Statistical analysis

The two-tailed, non-parametric Mann-Whitney and Kruskal-Wallis tests were used to test for statistically significant differences of independent samples in 2 or more groups, respectively. The non-parametric Wilcoxon test was employed to test paired samples. P values of less than 0.05 were considered significant. Calculations were performed using Medcalc statistical software (Medcalc, Mariakerke, Belgium).

### Example 1: Anti-glycoprotein 2 antibodies in patients with celiac disease are directed against the long isoforms 1 and 2

The experiments provided herein show that in serum samples obtained from CeD patients, significantly higher levels of IgG to the isoform 1, 2, and 3 of GP2 cGP2red with those in blood donors were observed. IgG reactivity to isoform 4 was not different (p > 0.05). In contrast, IgA levels to all four GP2 isoforms showed significantly higher levels in de-novo celiac disease patients than in controls. There was a significantly higher reactivity of IgG and IgA to the longer GP2 isoforms 1 and 2 of GP2 cGP2red with the shorter isoforms 3 and 4.

### Subjects

Ten serum samples from patients with de-novo CeD and 50 control sera from healthy blood donors (BD) were assessed. The median age of the 40 patients with CeD (8 females) was 16 years with an interquartile range (IQR) from 6 years to 22 years. The median age of the BD (23 females, 27 males) was 24 years (IQR 18 - 41). Clinical diagnoses were based upon standard clinical, radiological, endoscopic and histological criteria.

### Results

Patients with de-novo celiac disease demonstrated significantly higher levels of IgG to the isoform 1, 2, and 3 of GP2 cGP2red with those in blood donors (p = 0.001, p = 0.004, p = 0.0388 respectively) (fig. 2, 3). IgG reactivity to isoform 4 was not different (p > 0.05). In contrast, IgA levels to all 4 GP2 isoforms showed significantly higher levels in de-novo celiac disease patients than in controls (p < 0.001, respectively). There was a significantly higher reactivity of IgG and IgA to the longer GP2 isoforms 1 and 2 of GP2 cGP2red with the shorter isoforms 3 and 4 (p > 0.05, respectively).

Investigating patients with de-novo celiac disease and controls, receiver operating characteristics curve analysis revealed a significantly higher area under the curve (AUC) for IgG autoantibodies to isoform 1 (0.898, 95% CI: 0.792 - 0.961) and 2 (0.854, 95% CI: 0.739 - 0.932) cGP2red with autoantibodies to isoform 4 (0.638, 95% CI: 0.505 - 0.758, p < 0.05, respectively). There was a tendency for a higher AUC for IgG to isoform 1 and 2 in contrast to autoantibodies to isoform 3 (0.708, 95% CI: 0.576 - 0.818; p = 0.0725, p = 0.0652, respectively).

IgA autoantibodies to isoform 1 (1.000, 95% CI: 0.940 - 1.000) and 2 (1.000, 95% CI: 0.940 - 1.000) demonstrated a significantly higher AUC cGP2red with autoantibodies to isoform 3 (0.912, 95% CI: 0.810 - 0.970, p < 0.05, respectively). There was a tendency for a higher AUC for IgG to isoform 1 and 2 in contrast to autoantibodies to isoform 4 (0.957, 95% CI: 0.871 - 0.992; p = 0.0712, p = 0.0712, respectively).

In summary, autoantibodies to the longer isoforms of GP2 can be used for the diagnosis of de-novo celiac disease and are characterized by better assay performance characteristics than autoantibodies to the shorter isoforms.

### Example 2: Anti-glycoprotein 2 antibodies in patients with CD are directed against the long isoforms 1 and 2

The invention is also based on the finding that the GP2-isoforms as disclosed herein are surprisingly well suited for the diagnosis or therapy control of chronic inflammatory or other autoimmune diseases, especially Crohn's disease (CD) and Ulcerative colitis (UC). In particular, the novel GP2-isoforms as described herein allow a more reproducible and more accurate differnetiation between CD and UC. Autoantibodies to the shorter isoforms of GP2, in particular isoform 4, can be used for the differential diagnosis of inflammatory bowel diseases and show a better differentiation of Crohn's disease and ulcerative colitis patients.

### Subjects

44 patients with CD, 30 patients with CU and 21 blood donors were tested for anti-GP2 isotypes IgG and IgA. Clinical diagnoses were based upon standard clinical, radiological, endoscopic and histological criteria.

### Results

IgG to GP2 isoform 1 demonstrated significantly different OD values in 44 patients with CD, 30 patients with CU and 21 blood donors (Fig. 5, p < 0.0001). Patients with CD demonstrated elevated IgG to GP2 isoform 1 compared with those in patients with UC and blood donors (p < 0.05). Furthermore, patients with UC had higher antibodies to isoform 1 than blood donors did (p < 0.05).

Likewise IgG to GP2 isoforms 2 and 3 demonstrated also significantly different OD values in 44 patients with CD, 30 patients with CU and 21 blood donors (Fig. 6,7, p < 0.0001). Patients with CD showed elevated IgG to GP2 isoforms 2 and 3 compared with those in patients with UC and blood donors (p < 0.05, respectively). Furthermore, patients with UC had higher antibodies to isoform 2 and 3 than blood donors did (p < 0.05, respectively).

IgG to GP2 isoform 4 demonstrated also significantly different OD values in 44 patients with CD, 30 patients with CU and 21 blood donors (Fig. 8, p < 0.0001). Patients with CD revealed elevated IgG to GP2 isoform 4 compared with those in patients with UC and blood donors (p < 0.05). However, patients with UC did not have a different antibody level to isoform 4 compared with blood donors (p > 0.05).

IgA to GP2 isoforms 1,3 and 4 demonstrated also significantly different OD values in 44 patients with CD, 30 patients with CU and 21 blood donors (p < 0.0001). Patients with CD showed elevated IgA to GP2 isoforms 1,3 and 4 compared with those in patients with UC and blood donors (p < 0.05, respectively). However, patients with UC did not have a different antibody level those 3 isoforms compared with blood donors (p > 0.05).

IgA to GP2 isoform 2 demonstrated also significantly different OD values in 44 patients with CD, 30 patients with CU and 21 blood donors (p < 0.0001). Patients with CD revealed elevated IgG to GP2 isoform 2 compared with those in patients with UC (p < 0.05) and but not in blood donors (p > 0.05).

The results show that, through a receiver operating characteristic (ROC) analysis, the novel GP2-isoforms, in partiular GP2-isoform 4, enable an improved detection and/or discrimination for the serological diagnosis of Crohn's disease (CD) and Ulcerative colitis (CU).

The experiments disclosed herein demonstrate that clear differentiation between CD and UC is possible at the serological level by using the isoforms disclosed herein, preferably isoforms 3 and/or 4 of GP2 (Fig. 7 and 8). As can be observed in these figures, the IgG autoantibodies of UC patients do not bind the isoforms 3 and 4 of GP2. Therefore, the use of isoforms 3 and 4 in the diagnostic method as described herein will enable clear differentiation between CD and UC patients. #

### Abbreviations

BD, blood donor; CeD, celiac disease; Cl, confidence interval; CD or CrD, Crohn's disease; CV, coefficient of variation; ELISA, enzyme-linked immunosorbent assay; GFD, gluten-free diet; GP2, Glycoprotein 2; IBD, inflammatory bowel disease; IQR, interquartile range; M cell, micro-fold or membranous cell; PAB, pancreatic autoantibody; rho, Spearman's rank coefficient of correlation; RT, room temperature, UC Ulcerative colitis,

### Literature

1. Ronzio RA, Kronquist KE, Lewis DS, MacDonald RJ, Mohrlok SH, O'Donnell JJ, Jr.: Glycoprotein synthesis in the adult rat pancreas. IV. Subcellular distribution of membrane glycoproteins. Biochim Biophys Acta 1978, 508:65-84.
2. Roggenbuck D, Hausdorf G, Martinez-Gamboa L, Reinhold D, Buttner T, Jungblut PR, Porstmann T, Laass MW, Henker J, Buning C et al.: Identification of GP2, the major zymogen granule membrane glycoprotein, as the autoantigen of pancreatic antibodies in Crohn's disease. Gut 2009, 58:1620-1628.
3. Komorowski L, Teegen B, Probst C, Aulinger-Stocker K, Sina C, Fellermann K, Stocker W: Autoantibodies against exocrine pancreas in Crohn's disease are directed against two antigens: The glycoproteins CUZD1 and GP2. J Crohns Colitis 2012,pii: S1873-9946(12)00433-3. doi: 10.1016/j.crohns.2012.10.011. [Epub ahead of print].
4. Hase K, Kawano K, Nochi T, Pontes GS, Fukuda S, Ebisawa M, Kadokura K, Tobe T, Fujimura Y, Kawano S et al.: Uptake through glycoprotein 2 of FimH(+) bacteria by M cells initiates mucosal immune response. Nature 2009, 462:226-230.
5. Terahara K, Yoshida M, Igarashi O, Nochi T, Pontes GS, Hase K, Ohno H, Kurokawa S, Mejima M, Takayama N et al.: Comprehensive gene expression profiling of Peyer's patch M cells, villous M-like cells, and intestinal epithelial cells. J Immunol 2008, 180:7840-7846.
6. Werner L, Paclik D, Fritz C, Reinhold D, Roggenbuck D, Sturm A: Identification of pancreatic Glycoprotein 2 as an endogenous immunomodulator of innate and adaptive immune responses. J Immunol 2012, 189:2774-2783.
7. Holzl MA, Hofer J, Kovarik JJ, Roggenbuck D, Reinhold D, Goihl A, Gartner M, Steinberger P, Zlabinger GJ: The zymogen granule protein 2 (GP2) binds to scavenger receptor expressed on endothelial cells I (SREC-I). Cell Immunol 2011, 267:88-93.
8. Baumgart M, Dogan B, Rishniw M, Weitzman G, Bosworth B, Yantiss R, Orsi RH, Wiedmann M, McDonough P, Kim SG et al.: Culture independent analysis of ileal mucosa reveals a selective increase in invasive Escherichia coli of novel phylogeny relative to depletion of Clostridiales in Crohn's disease involving the ileum. ISME J 2007, 1:403-418.
9. Sollid LM: Coeliac disease: dissecting a complex inflammatory disorder. Nat Rev Immunol 2002, 2:647-655.
10. Tibble J, Sigthorsson G, Foster R, Sherwood R, Fagerhol M, Bjarnason I: Faecal calprotectin and faecal occult blood tests in the diagnosis of colorectal carcinoma and adenoma. Gut 2001, 49 2001, 49:402-408.
11. de KS, Keszthelyi D, Masclee AA: Leaky gut and diabetes mellitus: what is the link? Obes Rev 2011, 12:449-458.
12. Bossuyt X: Serologic markers in inflammatory bowel disease. Clin Chem 2006, 52:171-181.
13. Bonifacio E, Lampasona V, Genovese S, Ferrari M, Bosi E: Identification of protein tyrosine phosphatase-like IA2 (islet cell antigen 512) as the insulin-dependent diabetes-related 37/40K autoantigen and a target of islet-cell antibodies. J Immunol 1995, 155:5419-5426.
14. Baekkeskov S, Kanaani J, Jaume JC, Kash S: Does GAD have a unique role in triggering IDDM? J Autoimmun 2000, 15:279-286.
15. Conrad K, Schmechta H, Klafki A, Lobeck G, Uhlig HH, Gerdi S, Henker J: Serological differentiation of inflammatory bowel diseases. Eur J Gastroenterol Hepatol 2002, 14:129-135.
16. Bogdanos DP, Roggenbuck D, Reinhold D, Wex T, Pavlidis P, von Arnim U, Malfertheiner P, Forbes A, Conrad C, Laass M: Pancreatic-specific autoantibodies to glycoprotein 2 mirror disease location and behaviour in younger patients with Crohn's disease. BMC Gastroenterol 2012, 12:102.
17. Rieder F, Franke A, Dirmeier A, Lopez R, Lang S, Roggenbuck D, Rogler G, Klebl F: Mo1247 Serologic Anti-GP2 Antibodies Are Associated With Strictures and Need for Surgical Resection in Crohn's Disease. Gastroenterology 2013, 144:-S617.
18. Roggenbuck D, Reinhold D, Werner L, Schierack P, Bogdanos DP, Conrad K: Glycoprotein 2 antibodies in Crohn's disease. Adv Clin Chem 2013, 60:187-208.
19. Roggenbuck D, Reinhold D, Wex T, Goihl A, von Arnim U, Malfertheiner P, Buttner T, Porstmann T, Porstmann S, Liedvogel B et al.: Autoantibodies to GP2, the major zymogen granule membrane glycoprotein, are new markers in Crohn's disease. Clin Chim Acta 2011, 412:718-724.
20. Bogdanos DP, Rigopoulou EI, Smyk DS, Roggenbuck D, Reinhold D, Forbes A, Laass MW, Conrad K: Diagnostic value, clinical utility and pathogenic significance of reactivity to the molecular targets of Crohn's disease specific-pancreatic autoantibodies. Autoimmun Rev 2011, 11:143-148.
21. Op De BK, Vermeire S, Rutgeerts P, Bossuyt X: Antibodies to GP2, the major zymogen granule membrane glycoprotein, in inflammatory bowel diseases. Gut 2010.
22. Pavlidis P, Romanidou O, Roggenbuck D, Mytilinaiou M, Al-Sulttan F, Liaskos C, Smyk DS, Koutsoumpas A, Rigopoulou E, Conrad K et al.: Ileal inflammation may trigger the development of GP2-specific pancreatic autoantibodies in patients with crohn's disease. Clin Dev Immunol 2012, 2012:640835.
23. Somma V, Ababneh H, Ababneh A, Gatti S, Romagnoli V, Bendia E, Conrad K, Bogdanos DP, Roggenbuck D, Ciarrocchi G: The Novel Crohn's Disease Marker Anti-GP2 Antibody Is Associated with Ileocolonic Location of Disease. Gastroenterol Res Pract 2013, 2013:683824.
24. Roggenbuck D, Reinhold D, Schierack P, Bogdanos DP, Conrad K, Laass MW: Crohn's disease specific pancreatic antibodies: clinical and pathophysiological challenges. Clin Chem Lab Med 2013,1-12.
25. Bonaci-Nikolic B, Spuran M, Andrejevic S, Nikolic M: Autoantibodies to GP2, the major zymogen granule membrane glycoprotein, in patients with gluten-sensitive enteropathy: A possible serological trap. Clin Chim Acta 2012, 413:822-823.
26. Ludvigsson JF, Leffler DA, Bai JC, Biagi F, Fasano A, Green PHR, Hadjivassiliou M, Kaukinen K, Kelly CP, Leonard JN et al: The Oslo definitions for coeliac disease and related terms [abstract].2012, 1
27. Soderholm JD, Peterson KH, Olaison G, Franzen LE, Westrom B, Magnusson KE, Sjodahl R: Epithelial permeability to proteins in the noninflamed ileum of Crohn's disease? Gastroenterology 1999, 117:65-72.
28. Bjarnason I: Intestinal permeability. Gut 1994, 35:S18-S22.
29. Baumgart DC, Carding SR: Inflammatory bowel disease: cause and immunobiology. Lancet 2007, 369:1627-1640.
30. Rieder F, Lawrance IC, Leite A, Sans M: Predictors of fibrostenotic Crohn's disease. Inflamm Bowel Dis 2011, 17:2000-2007.
31. Bardella MT, Elli L, De MS, Floriani I, Torri V, Piodi L: Autoimmune disorders in patients affected by celiac sprue and inflammatory bowel disease. Ann Med 2009, 41:139-143.
32. Fasano A: Leaky gut and autoimmune diseases. Clin Rev Allergy Immunol 2012, 42:71-78.
33. Fukuoka S: Molecular cloning and sequences of cDNAs encoding alpha (large) and beta (small) isoforms of human pancreatic zymogen granule membrane-associated protein GP2. Biochim Biophys Acta 2000, 1491:376-380.
34. Conrad K, Roggenbuck D, Ittenson A, Reinhold D, Buettner T, Laass MW: A new dot immunoassay for simultaneous detection of celiac specific antibodies and IgA-deficiency. Clin Chem Lab Med 2011, 50:337-343.
35. Zöphel K, Wunderlich G, Kotzerke J, von Landenberg P, Roggenbuck D: M22 based (manual) ELISA for TSH-receptor antibody (TRAb) measurement is more sensitive than 2nd generation TRAb assays. Clin Chim Acta 2009, 403:266.

## Claims

1. In vitro method for the diagnosis of primary sclerosing cholangitis by detection of autoantibodies from a sample that bind to one or more isoforms of Glycoprotein 2 (GP2), comprising
- providing a sample of a subject exhibiting symptoms and/or suspected of having primary sclerosing cholangitis,
- providing two or more isoforms of Glycoprotein 2 (GP2), wherein at least one of isoforms 1 and/or 2 and at least one of isoforms 3 and/or 4 are provided, wherein the amino acid sequences of isoforms 1, 2, 3 and 4 are according to SEQ ID NO 1, 2, 3 and 4, respectively, or amino acid sequences of more than 80%, more than 85%, more than 90% or more preferably more than 95% sequence identity to sequences of SEQ ID NO 1, 2, 3 and 4,
- contacting said sample with said GP2 isoforms, and
- detecting autoantibodies from said sample that bind to said one or more isoforms
- wherein the contacting and detecting steps comprise: allowing autoantibodies in said sample to bind to more than one of said GP2 isoforms, thereby forming a complex (GP2-autoantibody complex), contacting said complex with a label to form a labeled complex; and detecting the presence or absence of the labeled complex for said GP2 isoforms, and associating the presence of the detected autoantibodies in the sample with the presence of primary sclerosing cholangitis.

2. Kit for the diagnosis of an autoimmune disorder by the detection of autoantibodies from a sample that bind to one or more isoforms of Glycoprotein 2 (GP2), comprising two or more isoforms of GP2), wherein at least one of isoforms 1 and/or 2 and at least one of isoforms 3 and/or 4 are present, wherein the amino acid sequences of isoforms 1, 2, 3 and 4 are according to SEQ ID NO 1, 2, 3 and 4, respectively, or amino acid sequences of more than 80%, more than 85%, more than 90% or more preferably more than 95% sequence identity to sequences of SEQ ID NO 1, 2, 3 and 4.

3. Kit for the diagnosis of an autoimmune disorder by the detection of autoantibodies from a sample that bind to one or more isoforms of Glycoprotein 2 (GP2) according to the preceding claim, comprising additionally:
- one or more human anti-immunoglobulin antibodies, wherein said human anti-immunoglobulin antibodies bind autoantibodies of Ig-subtypes IgG, IgA and/or IgM,
- a label, either capable of binding said human anti-Immunoglobulin antibody, or linked to said anti-Immunoglobulin antibody, and
- means for detecting said label, and
- a solid phase to which at least one of isoforms 1 and/or 2 and at least one of isoforms 3 and/or 4 are immobilized via a linker or spacer, or via binding or crosslinking, wherein the amino acid sequences of isoforms 1, 2, 3 and 4 are according to SEQ ID NO 1, 2, 3 and 4, respectively, or amino acid sequences of more than 80%, more than 85%, more than 90% or more preferably more than 95% sequence identity to sequences of SEQ ID NO 1, 2, 3 and 4.

## Patentansprüche

1. In-vitro-Verfahren zur Diagnose von primärer sklerosierender Cholangitis durch Detektion von Autoantikörpern in einer Probe, die an eine oder mehrere Isoformen des Glykoproteins 2 (GP2) binden, umfassend:
- Bereitstellen einer Probe von einem Subjekt, das Symptome der primären sklerosierenden Cholangitis zeigt und/oder bei dem diese vermutet wird,
- Bereitstellen von zwei oder mehr Isoformen des Glykoproteins 2 (GP2), wobei mindestens eine der Isoformen 1 und/oder 2 und mindestens eine der Isoformen 3 und/oder 4 bereitgestellt werden, wobei die Aminosäuresequenzen der Isoformen 1, 2, 3 und 4 jeweils den SEQ ID NO 1, 2, 3 und 4 entsprechen oder Aminosäuresequenzen, die mehr als 80 %, mehr als 85 %, mehr als 90 % oder vorzugsweise mehr als 95 % der Sequenzidentität der Sequenzen der SEQ ID NO 1, 2, 3 und 4 aufweisen,
- Kontaktieren der Probe mit den GP2-Isoformen und
- Detektion von Autoantikörpern in der Probe, die sich an eine oder mehrere der Isoformen binden,
- wobei die Schritte des Kontaktierens und Detektierens Folgendes umfassen: Zulassen, dass sich Autoantikörper in der Probe an mehr als eine der GP2-Isoformen binden, wodurch ein Komplex (GP2-Autoantikörper-Komplex) gebildet wird, Kontaktieren des Komplexes mit einem Marker, um einen markierten Komplex zu bilden; und Detektion des Vorliegens oder Nichtvorliegens des markierten Komplexes für die GP2-Isoformen und Zuordnen des Vorliegens der detektierten Autoantikörper in der Probe mit dem Vorliegen von primärer sklerosierender Cholangitis.

2. Kit zur Diagnose einer Autoimmunerkrankung durch Detektion von Autoantikörpern in einer Probe, die an eine oder mehrere Isoformen des Glykoproteins 2 (GP2) binden, umfassend zwei oder mehr Isoformen des GP2, wobei mindestens eine der Isoformen 1 und/oder 2 und mindestens eine der Isoformen 3 und/oder 4 vorhanden ist, wobei die Aminosäuresequenzen der Isoformen 1, 2, 3 und 4 jeweils den SEQ ID NO 1, 2, 3 und 4 entsprechen oder Aminosäuresequenzen, die mehr als 80 %, mehr als 85 %, mehr als 90 % oder vorzugsweise mehr als 95 % der Sequenzidentität der Sequenzen der SEQ ID NO 1, 2, 3 und 4 aufweisen.

3. Kit zur Diagnose einer Autoimmunerkrankung durch Detektion von Autoantikörpern in einer Probe, die an eine oder mehrere Isoformen des Glykoproteins 2 (GP2) binden, nach dem vorhergehenden Anspruch, das zusätzlich Folgendes umfasst:
- einen oder mehrere menschliche anti-Immunglobulin-Antikörper, wobei die menschlichen anti-Immunglobulin-Antikörper Autoantikörper der Ig-Subtypen IgG, IgA und/oder IgM binden,
- einen Marker, der entweder dazu in der Lage ist, den menschlichen anti-Immunglobulin-Antikörper zu binden oder mit dem anti-Immunglobulin-Antikörper verbunden ist, und
- Mittel zur Detektion des Markers und
- eine Festphase, an die mindestens eine der Isoformen 1 und/oder 2 und mindestens eine der Isoformen 3 und/oder 4 über einen Linker oder Spacer oder über eine Bindung oder Vernetzung immobilisiert sind, wobei die Aminosäuresequenzen der Isoformen 1, 2, 3 und 4 jeweils den SEQ ID NO 1, 2, 3 und 4 entsprechen oder Aminosäuresequenzen, die mehr als 80 %, mehr als 85 %, mehr als 90 % oder vorzugsweise mehr als 95 % der Sequenzidentität der Sequenzen der SEQ ID NO 1, 2, 3 und 4 aufweisen.

## Revendications

1. Procédé *in vitro* de diagnostic de cholangite sclérosante primaire par détection d'autoanticorps dans un échantillon qui se lient à un ou plusieurs isoformes de la glycoprotéine 2 (GP2), comprenant
- la fourniture d'un échantillon d'un sujet présentant des symptômes et/ou suspecté de souffrir de cholangite sclérosante primaire,
- la fourniture de deux isoformes ou plus de la glycoprotéine 2 (GP2), dans lequel au moins un des isoformes 1 et/ou 2 et au moins un des isoformes 3 et/ou 4 sont fournis, dans lequel les séquences d'acides aminés des isoformes 1, 2, 3 et 4 sont conformes à SEQ ID NO : 1, 2, 3 et 4, respectivement, ou des séquences d'acides aminés présentant une identité de plus de 80 %, de plus de 85 %, de plus de 90 % ou de préférence de plus de 95 % avec les séquences de SEQ ID NO : 1, 2, 3 et 4,
- la mise en contact dudit échantillon avec lesdits isoformes de GP2, et
- la détection d'auto-anticorps dans ledit échantillon qui se lient auxdits un ou plusieurs isoformes
- dans lequel les étapes de mise en contact et de détection comprennent : le fait de laisser les auto-anticorps dans ledit échantillon se lier à un ou plusieurs desdits isoformes de GP2, formant ainsi un complexe (complexe GP2-auto-anticorps), la mise en contact dudit complexe avec un marqueur pour former un complexe marqué ; et la détection de la présence ou de l'absence du complexe marqué pour lesdits isoformes de GP2, et l'association de la présence des autoanticorps détectés dans l'échantillon à la présence de la cholangite sclérosante primaire.

2. Kit de diagnostic d'un maladie auto-immun par la détection d'auto-anticorps dans un échantillon qui se lient à un ou plusieurs isoformes de la glycoprotéine 2 (GP2), comprenant deux isoformes ou plus de la GP2, dans lequel au moins un des isoformes 1 et/ou 2 et au moins un des isoformes 3 et/ou 4 sont présents, dans lequel les séquences d'acides aminés des isoformes 1, 2, 3 et 4 sont conformes à SEQ ID NO : 1, 2, 3 et 4, respectivement, ou des séquences d'acides aminés présentant une identité de plus de 80 %, de plus de 85 %, de plus de 90 % ou de préférence de plus de 95 % avec les séquences de SEQ ID NO : 1, 2, 3 et 4.

3. Kit de diagnostic d'un maladie auto-immun par la détection d'auto-anticorps dans un échantillon qui se lient à un ou plusieurs isoformes de la glycoprotéine 2 (GP2) selon la revendication précédente, comprenant en outre :
- un ou plusieurs anticorps anti-immunoglobuline humaine, dans lequel lesdits anticorps anti-immunoglobuline humaine se lient à des auto-anticorps de sous-type d'Ig IgG, IgA et/ou IgM,
- un marqueur, soit capable de se lier au dit anticorps anti-immunoglobuline humaine, soit lié au dit anticorps anti-immunoglobuline, et
- un moyen de détection dudit marqueur, et
- une phase solide à laquelle au moins un des isoformes 1 et/ou 2 et au moins un des isoformes 3 et/ou 4 sont immobilisés par l'intermédiaire d'un lieur ou d'un espaceur, ou par l'intermédiaire d'une liaison ou d'une réticulation, dans lequel les séquences d'acides aminés des isoformes 1, 2, 3 et 4 sont conformes à SEQ ID NO : 1, 2, 3 et 4 respectivement, ou des séquences d'acides aminés présentant une identité supérieure à 80 %, supérieure à 85 %, supérieure à 90 % ou de manière davantage préférée supérieure à 95 % aux séquences SEQ ID NO : 1, 2, 3 et 4.


### SEQUENCE LISTING

<110> GA Generic Assays
<120> GP2 ISOFORMS AND THEIR USE IN AUTOANTIBODY CAPTURE
<130> XII 2047/14EP1
<160> 8
<170> PatentIn version 3.5
<210> 1
   <211> 537
   <212> PRT
   <213> Homo sapiens
<400> 1
   Met Pro His Leu Met Glu Arg Met Val Gly Ser Gly Leu Leu Trp Leu
   1               5                   10                  15
   Ala Leu Val Ser Cys Ile Leu Thr Gln Ala Ser Ala Val Gln Arg Gly
               20                  25                  30
   Tyr Gly Asn Pro Ile Glu Ala Ser Ser Tyr Gly Leu Asp Leu Asp Cys
           35                  40                  45
   Gly Ala Pro Gly Thr Pro Glu Ala His Val Cys Phe Asp Pro Cys Gln
       50                  55                  60
   Asn Tyr Thr Leu Leu Asp Glu Pro Phe Arg Ser Thr Glu Asn Ser Ala
   65                  70                  75                  80
   Gly Ser Gln Gly Cys Asp Lys Asn Met Ser Gly Trp Tyr Arg Phe Val
                   85                  90                  95
   Gly Glu Gly Gly Val Arg Met Ser Glu Thr Cys Val Gln Val His Arg
               100                 105                 110
   Cys Gln Thr Asp Ala Pro Met Trp Leu Asn Gly Thr His Pro Ala Leu
           115                 120                 125
   Gly Asp Gly Ile Thr Asn His Thr Ala Cys Ala His Trp Ser Gly Asn
       130                 135                 140
   Cys Cys Phe Trp Lys Thr Glu Val Leu Val Lys Ala Cys Pro Gly Gly
   145                 150                 155                 160
   Tyr His Val Tyr Arg Leu Glu Gly Thr Pro Trp Cys Asn Leu Arg Tyr
                   165                 170                 175
   Cys Thr Val Pro Arg Asp Pro Ser Thr Val Glu Asp Lys Cys Glu Lys
               180                 185                 190
   Ala Cys Arg Pro Glu Glu Glu Cys Leu Ala Leu Asn Ser Thr Trp Gly
           195                 200                 205
   Cys Phe Cys Arg Gln Asp Leu Asn Ser Ser Asp Val His Ser Leu Gln
       210                 215                 220
   Pro Gln Leu Asp Cys Gly Pro Arg Glu Ile Lys Val Lys Val Asp Lys
   225                 230                 235                 240
   Cys Leu Leu Gly Gly Leu Gly Leu Gly Glu Glu Val Ile Ala Tyr Leu
                   245                 250                 255
   Arg Asp Pro Asn Cys Ser Ser Ile Leu Gln Thr Glu Glu Arg Asn Trp
               260                 265                 270
   Val Ser Val Thr Ser Pro Val Gln Ala Ser Ala Cys Arg Asn Ile Leu
           275                 280                 285
   Glu Arg Asn Gln Thr His Ala Ile Tyr Lys Asn Thr Leu Ser Leu Val
       290                 295                 300
   Asn Asp Phe Ile Ile Arg Asp Thr Ile Leu Asn Ile Asn Phe Gln Cys
   305                 310                 315                 320
   Ala Tyr Pro Leu Asp Met Lys Val Ser Leu Gln Ala Ala Leu Gln Pro
                   325                 330                 335
   Ile Val Ser Ser Leu Asn Val Ser Val Asp Gly Asn Gly Glu Phe Ile
               340                 345                 350
   Val Arg Met Ala Leu Phe Gln Asp Gln Asn Tyr Thr Asn Pro Tyr Glu
           355                 360                 365
   Gly Asp Ala Val Glu Leu Ser Val Glu Ser Val Leu Tyr Val Gly Ala
       370                 375                 380
   Ile Leu Glu Gln Gly Asp Thr Ser Arg Phe Asn Leu Val Leu Arg Asn
   385                 390                 395                 400
   Cys Tyr Ala Thr Pro Thr Glu Asp Lys Ala Asp Leu Val Lys Tyr Phe
                   405                 410                 415
   Ile Ile Arg Asn Ser Cys Ser Asn Gln Arg Asp Ser Thr Ile His Val
               420                 425                 430
   Glu Glu Asn Gly Gln Ser Ser Glu Ser Arg Phe Ser Val Gln Met Phe
   435 440 445
   Met Phe Ala Gly His Tyr Asp Leu Val Phe Leu His Cys Glu Ile His
   450 455 460
   Leu Cys Asp Ser Leu Asn Glu Gln Cys Gln Pro Ser Cys Ser Arg Ser
   465 470 475 480
   Gln Val Arg Ser Glu Val Pro Ala Ile Asp Leu Ala Arg Val Leu Asp
   485 490 495
   Leu Gly Pro Ile Thr Arg Arg Gly Ala Gln Ser Pro Gly Val Met Asn
   500 505 510
   Gly Thr Pro Ser Thr Ala Gly Phe Leu Val Ala Trp Pro Met Val Leu
   515 520 525
   Leu Thr Val Leu Leu Ala Trp Leu Phe
   530 535
<210> 2
   <211> 534
   <212> PRT
   <213> Homo sapiens
<400> 2
   Met Pro His Leu Met Glu Arg Met Val Gly Ser Gly Leu Leu Trp Leu
   1 5 10 15
   Ala Leu Val Ser Cys Ile Leu Thr Gln Ala Ser Ala Val Gln Arg Gly
   20 25 30
   Tyr Gly Asn Pro Ile Glu Ala Ser Ser Tyr Gly Leu Asp Leu Asp Cys
   35 40 45
   Gly Ala Pro Gly Thr Pro Glu Ala His Val Cys Phe Asp Pro Cys Gln
   50 55 60
   Asn Tyr Thr Leu Leu Asp Glu Pro Phe Arg Ser Thr Glu Asn Ser Ala
   65 70 75 80
   Gly Ser Gln Gly Cys Asp Lys Asn Met Ser Gly Trp Tyr Arg Phe Val
   85 90 95
   Gly Glu Gly Gly Val Arg Met Ser Glu Thr Cys Val Gln Val His Arg
   100 105 110
   Cys Gln Thr Asp Ala Pro Met Trp Leu Asn Gly Thr His Pro Ala Leu
           115                 120                 125
   Gly Asp Gly Ile Thr Asn His Thr Ala Cys Ala His Trp Ser Gly Asn
       130                 135                 140
   Cys Cys Phe Trp Lys Thr Glu Val Leu Val Lys Ala Cys Pro Gly Gly
   145                 150                 155                 160
   Tyr His Val Tyr Arg Leu Glu Gly Thr Pro Trp Cys Asn Leu Arg Tyr
                   165                 170                 175
   Cys Thr Asp Pro Ser Thr Val Glu Asp Lys Cys Glu Lys Ala Cys Arg
               180                 185                 190
   Pro Glu Glu Glu Cys Leu Ala Leu Asn Ser Thr Trp Gly Cys Phe Cys
           195                 200                 205
   Arg Gln Asp Leu Asn Ser Ser Asp Val His Ser Leu Gln Pro Gln Leu
       210                 215                 220
   Asp Cys Gly Pro Arg Glu Ile Lys Val Lys Val Asp Lys Cys Leu Leu
   225                 230                 235                 240
   Gly Gly Leu Gly Leu Gly Glu Glu Val Ile Ala Tyr Leu Arg Asp Pro
                   245                 250                 255
   Asn Cys Ser Ser Ile Leu Gln Thr Glu Glu Arg Asn Trp Val Ser Val
               260                 265                 270
   Thr Ser Pro Val Gln Ala Ser Ala Cys Arg Asn Ile Leu Glu Arg Asn
           275                 280                 285
   Gln Thr His Ala Ile Tyr Lys Asn Thr Leu Ser Leu Val Asn Asp Phe
       290                 295                 300
   Ile Ile Arg Asp Thr Ile Leu Asn Ile Asn Phe Gln Cys Ala Tyr Pro
   305                 310                 315                 320
   Leu Asp Met Lys Val Ser Leu Gln Ala Ala Leu Gln Pro Ile Val Ser
                   325                 330                 335
   Ser Leu Asn Val Ser Val Asp Gly Asn Gly Glu Phe Ile Val Arg Met
               340                 345                 350
   Ala Leu Phe Gln Asp Gln Asn Tyr Thr Asn Pro Tyr Glu Gly Asp Ala
           355                 360                 365
   Val Glu Leu Ser Val Glu Ser Val Leu Tyr Val Gly Ala Ile Leu Glu
   370 375 380
   Gln Gly Asp Thr Ser Arg Phe Asn Leu Val Leu Arg Asn Cys Tyr Ala
   385 390 395 400
   Thr Pro Thr Glu Asp Lys Ala Asp Leu Val Lys Tyr Phe Ile Ile Arg
   405 410 415
   Asn Ser Cys Ser Asn Gln Arg Asp Ser Thr Ile His Val Glu Glu Asn
   420 425 430
   Gly Gln Ser Ser Glu Ser Arg Phe Ser Val Gln Met Phe Met Phe Ala
   435 440 445
   Gly His Tyr Asp Leu Val Phe Leu His Cys Glu Ile His Leu Cys Asp
   450 455 460
   Ser Leu Asn Glu Gln Cys Gln Pro Ser Cys Ser Arg Ser Gln Val Arg
   465 470 475 480
   Ser Glu Val Pro Ala Ile Asp Leu Ala Arg Val Leu Asp Leu Gly Pro
   485 490 495
   Ile Thr Arg Arg Gly Ala Gln Ser Pro Gly Val Met Asn Gly Thr Pro
   500 505 510
   Ser Thr Ala Gly Phe Leu Val Ala Trp Pro Met Val Leu Leu Thr Val
   515 520 525
   Leu Leu Ala Trp Leu Phe
   530
<210> 3
   <211> 390
   <212> PRT
   <213> Homo sapiens
<400> 3
   Met Pro His Leu Met Glu Arg Met Val Gly Ser Gly Leu Leu Trp Leu
   1 5 10 15
   Ala Leu Val Ser Cys Ile Leu Thr Gln Ala Ser Ala Val Gln Arg Val
   20 25 30
   Pro Arg Asp Pro Ser Thr Val Glu Asp Lys Cys Glu Lys Ala Cys Arg
   35 40 45
   Pro Glu Glu Glu Cys Leu Ala Leu Asn Ser Thr Trp Gly Cys Phe Cys
       50                  55                  60
   Arg Gln Asp Leu Asn Ser Ser Asp Val His Ser Leu Gln Pro Gln Leu
   65                  70                  75                  80
   Asp Cys Gly Pro Arg Glu Ile Lys Val Lys Val Asp Lys Cys Leu Leu
                   85                  90                  95
   Gly Gly Leu Gly Leu Gly Glu Glu Val Ile Ala Tyr Leu Arg Asp Pro
               100                 105                 110
   Asn Cys Ser Ser Ile Leu Gln Thr Glu Glu Arg Asn Trp Val Ser Val
           115                 120                 125
   Thr Ser Pro Val Gln Ala Ser Ala Cys Arg Asn Ile Leu Glu Arg Asn
       130                 135                 140
   Gln Thr His Ala Ile Tyr Lys Asn Thr Leu Ser Leu Val Asn Asp Phe
   145                 150                 155                 160
   Ile Ile Arg Asp Thr Ile Leu Asn Ile Asn Phe Gln Cys Ala Tyr Pro
                   165                 170                 175
   Leu Asp Met Lys Val Ser Leu Gln Ala Ala Leu Gln Pro Ile Val Ser
               180                 185                 190
   Ser Leu Asn Val Ser Val Asp Gly Asn Gly Glu Phe Ile Val Arg Met
           195                 200                 205
   Ala Leu Phe Gln Asp Gln Asn Tyr Thr Asn Pro Tyr Glu Gly Asp Ala
       210                 215                 220
   Val Glu Leu Ser Val Glu Ser Val Leu Tyr Val Gly Ala Ile Leu Glu
   225                 230                 235                 240
   Gln Gly Asp Thr Ser Arg Phe Asn Leu Val Leu Arg Asn Cys Tyr Ala
                   245                 250                 255
   Thr Pro Thr Glu Asp Lys Ala Asp Leu Val Lys Tyr Phe Ile Ile Arg
               260                 265                 270
   Asn Ser Cys Ser Asn Gln Arg Asp Ser Thr Ile His Val Glu Glu Asn
           275                 280                 285
   Gly Gln Ser Ser Glu Ser Arg Phe Ser Val Gln Met Phe Met Phe Ala
           290                 295                 300
   Gly His Tyr Asp Leu Val Phe Leu His Cys Glu Ile His Leu Cys Asp
   305                 310                 315                 320
   Ser Leu Asn Glu Gln Cys Gln Pro Ser Cys Ser Arg Ser Gln Val Arg
                   325                 330                 335
   Ser Glu Val Pro Ala Ile Asp Leu Ala Arg Val Leu Asp Leu Gly Pro
               340                 345                 350
   Ile Thr Arg Arg Gly Ala Gln Ser Pro Gly Val Met Asn Gly Thr Pro
           355                 360                 365
   Ser Thr Ala Gly Phe Leu Val Ala Trp Pro Met Val Leu Leu Thr Val
       370                 375                 380
   Leu Leu Ala Trp Leu Phe
   385                 390
<210> 4
   <211> 387
   <212> PRT
   <213> Homo sapiens
<400> 4
   Met Pro His Leu Met Glu Arg Met Val Gly Ser Gly Leu Leu Trp Leu
   1               5                   10                  15
   Ala Leu Val Ser Cys Ile Leu Thr Gln Ala Ser Ala Val Gln Arg Asp
               20                  25                  30
   Pro Ser Thr Val Glu Asp Lys Cys Glu Lys Ala Cys Arg Pro Glu Glu
           35                  40                  45
   Glu Cys Leu Ala Leu Asn Ser Thr Trp Gly Cys Phe Cys Arg Gln Asp
       50                  55                  60
   Leu Asn Ser Ser Asp Val His Ser Leu Gln Pro Gln Leu Asp Cys Gly
   65                  70                  75                  80
   Pro Arg Glu Ile Lys Val Lys Val Asp Lys Cys Leu Leu Gly Gly Leu
                   85                  90                  95
   Gly Leu Gly Glu Glu Val Ile Ala Tyr Leu Arg Asp Pro Asn Cys Ser
               100                 105                 110
   Ser Ile Leu Gln Thr Glu Glu Arg Asn Trp Val Ser Val Thr Ser Pro
           115                 120                 125
   Val Gln Ala Ser Ala Cys Arg Asn Ile Leu Glu Arg Asn Gln Thr His
       130                 135                 140
   Ala Ile Tyr Lys Asn Thr Leu Ser Leu Val Asn Asp Phe Ile Ile Arg
   145                 150                 155                 160
   Asp Thr Ile Leu Asn Ile Asn Phe Gln Cys Ala Tyr Pro Leu Asp Met
                   165                 170                 175
   Lys Val Ser Leu Gln Ala Ala Leu Gln Pro Ile Val Ser Ser Leu Asn
               180                 185                 190
   Val Ser Val Asp Gly Asn Gly Glu Phe Ile Val Arg Met Ala Leu Phe
           195                 200                 205
   Gln Asp Gln Asn Tyr Thr Asn Pro Tyr Glu Gly Asp Ala Val Glu Leu
       210                 215                 220
   Ser Val Glu Ser Val Leu Tyr Val Gly Ala Ile Leu Glu Gln Gly Asp
   225                 230                 235                 240
   Thr Ser Arg Phe Asn Leu Val Leu Arg Asn Cys Tyr Ala Thr Pro Thr
                   245                 250                 255
   Glu Asp Lys Ala Asp Leu Val Lys Tyr Phe Ile Ile Arg Asn Ser Cys
               260                 265                 270
   Ser Asn Gln Arg Asp Ser Thr Ile His Val Glu Glu Asn Gly Gln Ser
           275                 280                 285
   Ser Glu Ser Arg Phe Ser Val Gln Met Phe Met Phe Ala Gly His Tyr
       290                 295                 300
   Asp Leu Val Phe Leu His Cys Glu Ile His Leu Cys Asp Ser Leu Asn
   305                 310                 315                 320
   Glu Gln Cys Gln Pro Ser Cys Ser Arg Ser Gln Val Arg Ser Glu Val
                   325                 330                 335
   Pro Ala Ile Asp Leu Ala Arg Val Leu Asp Leu Gly Pro Ile Thr Arg
               340                 345                 350
   Arg Gly Ala Gln Ser Pro Gly Val Met Asn Gly Thr Pro Ser Thr Ala
           355                 360                 365
   Gly Phe Leu Val Ala Trp Pro Met Val Leu Leu Thr Val Leu Leu Ala
   370 375 380
   Trp Leu Phe
   385
<210> 5
   <211> 1614
   <212> DNA
   <213> Homo sapiens
<400> 5
   atgcctcacc ttatggaaag gatggtgggc tctggcctcc tgtggctggc cttggtctcc 60
   tgcattctga cccaggcatc tgcagtgcag cgaggttatg gaaaccccat tgaagccagt 120
   tcgtatgggc tggacctgga ctgcggagct cctggcaccc cagaggctca tgtctgtttt 180
   gacccctgtc agaattacac cctcctggat gaacccttcc gaagcacaga gaactcagca 240
   gggtcccagg ggtgcgataa aaacatgagc ggctggtacc gctttgtagg ggaaggagga 300
   gtaaggatgt cggagacctg tgtccaggtg caccgatgcc agacagacgc tcccatgtgg 360
   ctgaatggga cccaccctgc ccttggggat ggcatcacca accacactgc ctgtgcccat 420
   tggagtggca actgctgttt ctggaaaaca gaggtgctgg tgaaggcctg cccaggcggg 480
   taccatgtgt accggttgga aggcactccc tggtgtaatc tgagatactg cacagttcca 540
   cgagacccat ccactgtgga ggacaagtgt gagaaggcct gccgccccga ggaggagtgc 600
   cttgccctca acagcacctg gggctgtttc tgcagacagg acctcaatag ttctgatgtc 660
   cacagtttgc agcctcagct agactgtggg cccagggaga tcaaggtgaa ggtggacaaa 720
   tgtttgctgg gaggcctggg tttgggggag gaggtcattg cctacctgcg agacccaaac 780
   tgcagcagca tcttgcagac agaggagagg aactgggtat ctgtgaccag ccccgtccag 840
   gctagtgcct gcaggaacat tctggagaga aatcaaaccc atgccatcta caaaaacacc 900
   ctctccttgg tcaatgattt catcatcaga gacaccatcc tcaacatcaa cttccaatgt 960
   gcctacccac tggacatgaa agtcagcctc caagctgcct tgcagcccat tgtaagttcc 1020
   ctgaacgtca gtgtggacgg gaatggagag ttcattgtca ggatggccct cttccaagac 1080
   cagaactaca cgaatcctta cgaaggggat gcagttgaac tgtctgttga gtccgtgctg 1140
   tatgtgggtg ccatcttgga acaaggggac acctcccggt ttaacctggt gttgaggaac 1200
   tgctatgcca cccccactga agacaaggct gaccttgtga agtatttcat catcagaaac 1260
   agctgctcaa atcaacgtga ttccaccatc cacgtggagg agaatgggca gtcctcggaa 1320
   agccggttct cagttcagat gttcatgttt gctggacatt atgacctagt tttcctgcat 1380
   tgtgagattc atctctgtga ttctcttaat gaacagtgcc agccttcttg ctcaagaagt 1440
   caagtccgca gtgaagtacc ggccatcgac ctagcccggg ttctagattt ggggcccatc 1500
   actcggagag gtgcacagtc tcccggtgtc atgaatggaa cccctagcac tgcagggttc 1560
   ctggtggcct ggcctatggt cctcctgact gtcctcctgg cttggctgtt ctga 1614
<210> 6
   <211> 1605
   <212> DNA
   <213> Homo sapiens
<400> 6
   atgcctcacc ttatggaaag gatggtgggc tctggcctcc tgtggctggc cttggtctcc 60
   tgcattctga cccaggcatc tgcagtgcag cgaggttatg gaaaccccat tgaagccagt 120
   tcgtatgggc tggacctgga ctgcggagct cctggcaccc cagaggctca tgtctgtttt 180
   gacccctgtc agaattacac cctcctggat gaacccttcc gaagcacaga gaactcagca 240
   gggtcccagg ggtgcgataa aaacatgagc ggctggtacc gctttgtagg ggaaggagga 300
   gtaaggatgt cggagacctg tgtccaggtg caccgatgcc agacagacgc tcccatgtgg 360
   ctgaatggga cccaccctgc ccttggggat ggcatcacca accacactgc ctgtgcccat 420
   tggagtggca actgctgttt ctggaaaaca gaggtgctgg tgaaggcctg cccaggcggg 480
   taccatgtgt accggttgga aggcactccc tggtgtaatc tgagatactg cacagaccca 540
   tccactgtgg aggacaagtg tgagaaggcc tgccgccccg aggaggagtg ccttgccctc 600
   aacagcacct ggggctgttt ctgcagacag gacctcaata gttctgatgt ccacagtttg 660
   cagcctcagc tagactgtgg gcccagggag atcaaggtga aggtggacaa atgtttgctg     720
   ggaggcctgg gtttggggga ggaggtcatt gcctacctgc gagacccaaa ctgcagcagc     780
   atcttgcaga cagaggagag gaactgggta tctgtgacca gccccgtcca ggctagtgcc 840
   tgcaggaaca ttctggagag aaatcaaacc catgccatct acaaaaacac cctctccttg 900
   gtcaatgatt tcatcatcag agacaccatc ctcaacatca acttccaatg tgcctaccca 960
   ctggacatga aagtcagcct ccaagctgcc ttgcagccca ttgtaagttc cctgaacgtc 1020
   agtgtggacg ggaatggaga gttcattgtc aggatggccc tcttccaaga ccagaactac 1080
   acgaatcctt acgaagggga tgcagttgaa ctgtctgttg agtccgtgct gtatgtgggt 1140
   gccatcttgg aacaagggga cacctcccgg tttaacctgg tgttgaggaa ctgctatgcc 1200
   acccccactg aagacaaggc tgaccttgtg aagtatttca tcatcagaaa cagctgctca 1260
   aatcaacgtg attccaccat ccacgtggag gagaatgggc agtcctcgga aagccggttc 1320
   tcagttcaga tgttcatgtt tgctggacat tatgacctag ttttcctgca ttgtgagatt 1380
   catctctgtg attctcttaa tgaacagtgc cagccttctt gctcaagaag tcaagtccgc 1440
   agtgaagtac cggccatcga cctagcccgg gttctagatt tggggcccat cactcggaga 1500
   ggtgcacagt ctcccggtgt catgaatgga acccctagca ctgcagggtt cctggtggcc 1560
   tggcctatgg tcctcctgac tgtcctcctg gcttggctgt tctga 1605
<210> 7
   <211> 1173
   <212> DNA
   <213> Homo sapiens
<400> 7
   atgcctcacc ttatggaaag gatggtgggc tctggcctcc tgtggctggc cttggtctcc    60
   tgcattctga cccaggcatc tgcagtgcag cgagttccac gagacccatc cactgtggag   120
   gacaagtgtg agaaggcctg ccgccccgag gaggagtgcc ttgccctcaa cagcacctgg   180
   ggctgtttct gcagacagga cctcaatagt tctgatgtcc acagtttgca gcctcagcta   240
   gactgtgggc ccagggagat caaggtgaag gtggacaaat gtttgctggg aggcctgggt   300
   ttgggggagg aggtcattgc ctacctgcga gacccaaact gcagcagcat cttgcagaca   360
   gaggagagga actgggtatc tgtgaccagc cccgtccagg ctagtgcctg caggaacatt   420
   ctggagagaa atcaaaccca tgccatctac aaaaacaccc tctccttggt caatgatttc   480
   atcatcagag acaccatcct caacatcaac ttccaatgtg cctacccact ggacatgaaa   540
   gtcagcctcc aagctgcctt gcagcccatt gtaagttccc tgaacgtcag tgtggacggg   600
   aatggagagt tcattgtcag gatggccctc ttccaagacc agaactacac gaatccttac   660
   gaaggggatg cagttgaact gtctgttgag tccgtgctgt atgtgggtgc catcttggaa   720
   caaggggaca cctcccggtt taacctggtg ttgaggaact gctatgccac ccccactgaa   780
   gacaaggctg accttgtgaa gtatttcatc atcagaaaca gctgctcaaa tcaacgtgat   840
   tccaccatcc acgtggagga gaatgggcag tcctcggaaa gccggttctc agttcagatg   900
   ttcatgtttg ctggacatta tgacctagtt ttcctgcatt gtgagattca tctctgtgat   960
   tctcttaatg aacagtgcca gccttcttgc tcaagaagtc aagtccgcag tgaagtaccg  1020
   gccatcgacc tagcccgggt tctagatttg gggcccatca ctcggagagg tgcacagtct  1080
   cccggtgtca tgaatggaac ccctagcact gcagggttcc tggtggcctg gcctatggtc  1140
   ctcctgactg tcctcctggc ttggctgttc tga                              1173
<210> 8
   <211> 1164
   <212> DNA
   <213> Homo sapiens
<400> 8
   atgcctcacc ttatggaaag gatggtgggc tctggcctcc tgtggctggc cttggtctcc    60
   tgcattctga cccaggcatc tgcagtgcag cgagacccat ccactgtgga ggacaagtgt   120
   gagaaggcct gccgccccga ggaggagtgc cttgccctca acagcacctg gggctgtttc   180
   tgcagacagg acctcaatag ttctgatgtc cacagtttgc agcctcagct agactgtggg      240
   cccagggaga tcaaggtgaa ggtggacaaa tgtttgctgg gaggcctggg tttgggggag      300
   gaggtcattg cctacctgcg agacccaaac tgcagcagca tcttgcagac agaggagagg      360
   aactgggtat ctgtgaccag ccccgtccag gctagtgcct gcaggaacat tctggagaga      420
   aatcaaaccc atgccatcta caaaaacacc ctctccttgg tcaatgattt catcatcaga      480
   gacaccatcc tcaacatcaa cttccaatgt gcctacccac tggacatgaa agtcagcctc      540
   caagctgcct tgcagcccat tgtaagttcc ctgaacgtca gtgtggacgg gaatggagag      600
   ttcattgtca ggatggccct cttccaagac cagaactaca cgaatcctta cgaaggggat      660
   gcagttgaac tgtctgttga gtccgtgctg tatgtgggtg ccatcttgga acaaggggac      720
   acctcccggt ttaacctggt gttgaggaac tgctatgcca cccccactga agacaaggct      780
   gaccttgtga agtatttcat catcagaaac agctgctcaa atcaacgtga ttccaccatc      840
   cacgtggagg agaatgggca gtcctcggaa agccggttct cagttcagat gttcatgttt      900
   gctggacatt atgacctagt tttcctgcat tgtgagattc atctctgtga ttctcttaat      960
   gaacagtgcc agccttcttg ctcaagaagt caagtccgca gtgaagtacc ggccatcgac     1020
   ctagcccggg ttctagattt ggggcccatc actcggagag gtgcacagtc tcccggtgtc     1080
   atgaatggaa cccctagcac tgcagggttc ctggtggcct ggcctatggt cctcctgact     1140
   gtcctcctgg cttggctgtt ctga                                            1164